(19) Europäisches Patentamt
European Patent Office
Office européen des brevets

(11) Veröffentlichungsnummer: **0 609 259 B1**

# (12) EUROPÄISCHE PATENTSCHRIFT

(45) Veröffentlichungstag der Patentschrift: **22.11.95**

(51) Int. Cl.[6]: **C07C 69/757**, C07C 251/42, C07C 251/18, A01N 37/42, C07C 67/313

(21) Anmeldenummer: **92920691.0**

(22) Anmeldetag: **26.09.92**

(86) Internationale Anmeldenummer: **PCT/EP92/02226**

(87) Internationale Veröffentlichungsnummer: **WO 93/08153 (29.04.93 93/11)**

## (54) CYCLOHEXENONDERIVATE ALS HERBIZIDE.

(30) Priorität: **25.10.91 DE 4135265**

(43) Veröffentlichungstag der Anmeldung: **10.08.94 Patentblatt 94/32**

(45) Bekanntmachung des Hinweises auf die Patenterteilung: **22.11.95 Patentblatt 95/47**

(84) Benannte Vertragsstaaten: **AT BE CH DE ES FR GB IT LI NL**

(56) Entgegenhaltungen:
**EP-A- 0 126 713**
**EP-A- 0 306 996**

(73) Patentinhaber: **BASF Aktiengesellschaft**
**Carl-Bosch-Strasse 38**
**D-67063 Ludwigshafen (DE)**

(72) Erfinder: **KAST, Juergen**
**Forststrasse 31**
**D-6737 Boehl-Iggelheim (DE)**
Erfinder: **ZIERKE, Thomas**
**Akazienstrasse 12**
**D-6737 Boehl-Iggelheim (DE)**
Erfinder: **BRATZ, Matthias**
**Schwabsgasse 2**
**D-6720 Speyer (DE)**
Erfinder: **MISSLITZ, Ulf**
**Am Herzel 40**
**D-6730 Neustadt (DE)**
Erfinder: **MEYER, Norbert**
**Dossenheimer Weg 22**
**D-6802 Ladenburg (DE)**
Erfinder: **LANDES, Andreas**
**Untere Hart 12**
**D-6703 Limburgerhof (DE)**
Erfinder: **RADEMACHER, Wilhelm**
**Austrasse 1**
**D-6703 Limburgerhof (DE)**
Erfinder: **WESTPHALEN, Karl-Otto**
**Mausbergweg 58**
**D-6720 Speyer (DE)**
Erfinder: **WALTER, Helmut**
**Gruenstadter Strasse 82**
**D-6719 Obrigheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 609 259 B1

## Beschreibung

Die vorliegende Erfindung betrifft neue Cyclohexenonderivate der allgemeinen Formel I

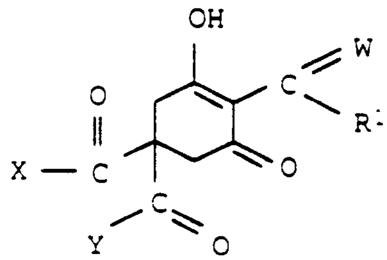

I

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe gewünschtenfalls noch ein- bis drei Reste tragen können, ausgewahlt aus einer Gruppe bestehend aus Nitro, Cyano, Halogen, $C_1$-$C_4$-Alkyl, partiell oder vollständig halogeniertem $C_1$-$C_4$-Alkyl, $C_1$-$C_4$-Alkoxy, $C_1$-$C_4$-Alkylsulfinyl und $C_1$-$C_4$-Alkylsulfonyl;

W Sauerstoff, eine Gruppe $=N-OR^2$ oder $=N-R^3$, mit

$R^2$

- einen $C_1$-$C_6$-Alkyl-, $C_3$-$C_6$-Alkenyl- oder $C_3$-$C_6$-Alkenylrest, wobei diese Reste noch einen bis drei der folgen den Substituenten tragen können, ausgewahlt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewahlt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl,partiell oder vollständig halogeniertem $C_1$-$C_4$-Alkyl, $C_1$-$C_4$-Alkoxy und partiell oder vollstandig halogeniertem $C_1$-$C_4$-Alkoxy;
- eine 3- bis 6-gliedrige Alkylkette oder eine 4- bis 6-gliedrige Alkenyl- oder Alkinylkette, wobei jeweils ein Kettenglied durch Sauerstoff, Schwefel oder eine Gruppe -SO-, $-SO_2$- oder $-N(R^8)$- ersetzt ist, und wobei die Kette noch zusätzlich ein bis drei Substituenten tragen kann, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, partiell oder vollstandig halogeniertem $C_1$-$C_4$-Alkyl, $C_1$-$C_4$-Alkoxy und partiell oder vollständig halogeniertem $C_1$-$C_4$-Alkoxy;

$R^8$ steht für Wasserstoff, $C_1$-$C_6$-Alkyl, $C_1$-$C_6$-Alkenyl, $C_1$-$C_6$-Alkinyl, $C_1$-$C_6$-Alkylcarbonyl oder Benzoyl;

$R^3$

Wasserstoff, einen $C_1$-$C_6$-Alkylrest, einen Hydroxy-$C_1$-$C_4$-alkyl-, $C_1$-$C_4$-Alkoxy-$C_1$-$C_2$-alkyl- oder $C_1$-$C_4$-Alkylthio-$C_1$-$C_2$-alkylrest, den Phenyl- oder Benzylrest, wobei die aromatischen Ringe noch ein bis drei Substituenten tragen können, ausgewählt aus einer Gruppe bestehend aus Nitro, Halogen, $C_1$-$C_4$-Alkyl, partiell oder vollständig halogeniertem $C_1$-$C_4$-Alkyl und $C_1$-$C_4$-Alkoxy;

X,Y

eine Gruppe $-OR^4$ oder $-NR^5R^6$, wobei

$R^4$

Wasserstoff, einen $C_1$-$C_6$-Alkyl-, $C_3$-$C_6$-Alkenyl-, $C_3$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-

Alkoxy-$C_1$-$C_4$-alkyl- oder $C_1$-$C_4$-Alkylthio-$C_1$-$C_4$-alkylrest, und

$R^6$ Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, partiell oder vollständig halogeniertem $C_1$-$C_4$-Alkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe -N($R^7$)- als Ringglied enthalten kann, wobei

$R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht,

bedeuten,

sowie ihre landwirtschaftlich brauchbaren Salze und Ester von $C_1$-$c_{10}$-Carbonsäuren und anorganischen Säuren.

Außerdem betrifft die Erfindung Verfahren zur Herstellung dieser Verbindungen, ihre Verwendung als Herbizide und zur Regulierung des Pflanzenwachstums sowie herbizide Mittel und Mittel zur Regulierung des Pflanzenwachstums, welche diese Verbindungen als wirksame Substanzen enthalten.

Aus der Literatur sind herbizide wirksame Cyclohexenonderivate,die in 2-Stellung eine Oximethergruppe tragen, bekannt (US 4,249,937; CA 1,205,813; Adv. Pest. Science, Part 2, Pergamon Press, Zürich, 1978; E.H. Geissbühler, Proc. 4th Inern. Congress of Pesticide Chemistry (IUPAC), 1978, 235; EP-A-0 368 227, DE-A-40 14 983, DE-A-40 14 984, DE-A-40 14 986, DE-A-40 14 987 und DE-A-40 14 988).

Des weiteren ist bekannt, daß bestimmte 2-Acyl-3-hydroxycyclohex-2-en-1-one regulierend auf das Pflanzenwachstum einwirken (US 4,560,403, US 4,584,013, US 4,640,706, EP-A-177 450, EP-A-199 658, EP-A-293 817).

Aus den EP-A 123 001, 126 713 und 338 525 ist bekannt, da Cyclohexantrione mit einer Alkoxycarbonyl- oder Dialkylaminocarbonylgruppe am Cyclohexandionring wachstumsretardierende Eigenschaften aufweisen.

Außerdem sind aus den Druckschriften BE 833 488, J5 7045143, J5 1013750, J5 7046943, J5 4016454, J5 1131856, DE-A-24 61 027, DE-A-24 39 104 und US 4,011,256 Cyclohexenonoximether, mit einer Alkoxycarbonyl oder Dialkylaminocarbonylgruppe am Cyclohexandionring, bekannt, die herbizide Eigenschaften aufweisen.

Die herbiziden und das Wachstum der Pflanzen regulierenden Eigenschaften dieser Verbindungen können jedoch, besonders bei kleinen Aufwandmengen und -konzentrationen, nur bedingt befriedigen.

Die in der Definition der Substituenten $R^1$ bis $R^8$ verwendeten Sammelbegriffe

- Halogen,
- $C_1$-$C_6$-Alkyl, $C_1$-$C_4$-Alkyl, $C_2$-$C_6$-Alkenyl, $C_3$-$C_6$-Alkenyl, $C_2$-$C_6$-Alkinyl, $C_3$-$C_6$-Alkinyl,
- partiell oder vollständig halogeniertes $C_1$-$C_4$-Alkyl, partiell oder vollständig halogeniertes $C_1$-$C_4$-Alkoxy,
- $C_1$-$C_4$-Alkoxy, $C_1$-$C_4$-Alkylthio, $C_1$-$C_6$-Alkylcarbonyl,
- $C_1$-$C_4$-Alkoxy-$C_1$-$C_4$-alkyl, $C_1$-$C_4$-Alkylthio-$C_1$-$C_4$-alkyl,
- $C_1$-$C_4$-Alkylsulfinyl, $C_1$-$C_4$-Alkylsulfonyl und
- 5- oder 6-gliedriges Heteroaryl mit ein oder zwei Heteroatomen, ausgewählt aus einer Gruppe bestehend aus Stickstoff, Sauerstoff und Schwefel,

stellen Kurzschreibweisen für eine individuelle Aufzählung der einzelnen Gruppenmitglieder dar. Sämtliche Alkyl-, Alkenyl-, Alkinyl-, Halogenalkyl- bzw. -alkoxyteile können geradkettig oder verzweigt sein. Die partiell oder vollständig halogenierten Alkyl- bzw. Alkoxyteile können gleiche oder verschiedene Halogenatome tragen. Im einzelnen bedeuten beispielsweise:

- Halogen: Fluor, Chlor, Brom und Jod, vorzugsweise Fluor und Chlor;
- $C_1$-$C_6$-Alkyl: Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Isopropyl, n-Butyl und tert.-Butyl;
- $C_1$-$C_4$-Alkyl: Methyl, Ethyl, n-Propyl, Isopropyl, n-Butyl und tert.-Butyl;

- $C_2$-$C_6$-Alkenyl: Vinyl und $C_3$-$C_6$-Alkenyl wie Prop-1-en-1-yl, Prop-2-en-1-yl, 1-Methylethenyl, n-But-1-en-1-yl, n-But-1-en-2-yl, n-But-1-en-3-yl, 1-Methyl-prop-1-en-1-yl, 2-Methyl-prop-1-en-1-yl, 1-Methyl-prop-2-en-1-yl, 2-Methyl-prop-2-en-1-yl, n-Pent-1-en-1-yl, n-Pent-1-en-2-yl, n-Pent-1-en-3-yl, n-Pent-1-en-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl, vorzugsweise Prop-2-en-1-yl und But-2-en-1-yl;
- $C_2$-$C_6$-Alkinyl: Ethinyl und $C_3$-$C_6$-Alkinyl wie Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl, vorzugsweise Prop-2-in-1-yl;
- $C_3$-$C_6$-Cycloalkyl: Cyclopropyl, Cyclobutyl, Cyclopentyl und Cyclohexyl, vorzugsweise Cyclopropyl, Cyclopentyl und Cyclohexyl;
- partiell oder vollständig halogeniertes $C_1$-$C_4$-Alkyl: Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl und 3-Chlorpropyl, vorzugsweise Trifluormethyl;
- Hydroxy-($C_1$-$C_4$)-alkyl: Hydroxymethyl, 1-Hydroxyeth-1-yl, 2-Hydroxyeth-1-yl, 1-Hydroxy-prop-1-yl, 2-Hydroxy-prop-1-yl, 3-Hydroxy-prop-1-yl, 1-Hydroxy-prop-2-yl, 2-Hydroxy-prop-2-yl, 1-Hydroxy-but-1-yl, 2-Hydroxy-but-1-yl, 3-Hydroxy-but-1-yl, 4-Hydroxy-but-1-yl, 1-Hydroxy-but-2-yl, 2-Hydroxy-but-2-yl, 1-Hydroxy-but-3-yl, 2-Hydroxy-but-3-yl, 1-Hydroxy-2-methyl-prop-3-yl, 2-Hydroxy-2-methyl-prop-3-yl, 3-Hydroxy-2-methyl-prop-3-yl, und 2-Hydroxymethyl-prop-2-yl;
- Phenyl-$C_1$-$C_4$-alkyl: Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl, und 1-(Phenylmethyl)-prop-1-yl;
- $C_1$-$C_4$-Alkoxy, Methoxy, Ethoxy, n-Propoxy, 1-Methylethoxy, n-Butoxy, 1-Methyl-propoxy, 2-Methyl-propoxy und 1,1-Dimethylethoxy, vorzugsweise Methoxy und Ethoxy;
- partiell oder vollständig halogeniertes $C_1$-$C_4$-Alkoxy: Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, vorzugsweise Trifluormethoxy;
- $C_1$-$C_4$-Alkylthio: Methylthio, Ethylthio, n-Propylthio, Isopropylthio, n-Butylthio und tert.-Butylthio, vorzugsweise Methylthio und Ethylthio;
- $C_1$-$C_4$-Alkylthio-$C_1$-$C_4$-alkyl: Methylthiomethyl, Ethylthiomethyl, n-Propylthiomethyl, (1-Methylethylthio)methyl, n-Butylthiomethyl, (1-Methylpropylthio)methyl, (2-Methylpropylthio)methyl, (1,1-Dimethylethylthio)methyl, Methylthioethyl, Ethylthioethyl, n-Propylthioethyl, (1-Methylethylthio)ethyl, n-Butylthioethyl, (1-Methylpropylthio)ethyl, (2-Methylpropylthio)ethyl, (1,1-Dimethylethylthio)-ethyl, 3-(Methylthio)propyl, 2-(Methylthio)propyl und 2-(Ethylthio)-propyl, vorzugsweise Methylthiomethyl, Ethylthiomethyl, 2-Methylthioethyl und 2-Ethylthioethyl;
- $C_1$-$C_4$-Alkoxy-$C_1$-$C_4$-alkyl: Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, Methoxyethyl, Ethoxyethyl, n-Propoxyethyl, (1-Methylethoxy)ethyl, n-Butoxyethyl, (1-Methylpropoxy)ethyl,

(2-Methylpropoxy)ethyl, (1,1-Dimethylethoxy)ethyl, 3-(Methoxy)propyl, 2-(Methoxy)propyl und 2-(Ethoxy)propyl, vorzugsweise Methoxymethyl, Ethoxymethyl, 2-Methoxyethyl und 2-Ethoxyethyl;

- $C_1$-$C_4$-Alkylcarbonyl: Methylcarbonyl, Ethylcarbonyl, n-Propylcarbonyl, 1-Methylethyl-carbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methyl-propylcarbonyl und 1-Ethyl-2-methyl-propylcarbonyl, vorzugsweise Methylcarbonyl, Ethylcarbonyl, n-Propylcarbonyl und 1,1-Dimethylethylcarbonyl;
- $C_1$-$C_4$-Alkylsulfinyl: Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl und 1,1-Dimethylethylsulfinyl, vorzugsweise Methyl- und Ethylsulfinyl;
- $C_1$-$C_4$-Alkylsulfonyl: Methylsulfonyl, Ethylsulfonyl, n-Propylsulfonyl, 1-Methylethyl-sulfonyl, n-Butylsulfonyl, 1-Methylpropylsulfonyl, 2-Methylpropylsulfonyl und 1,1-Dimethylethylsulfonyl, vorzugsweise Methyl- und Ethylsulfonyl;
- ein 5- oder 6-gliedriger aromatischer Heterocyclus mit ein oder zwei Heteroaromaten, ausgewählt aus einer Gruppe bestehend aus Stickstoff, Sauerstoff und Schwefel: 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Im Hinblick auf die biologische Wirksamkeit werden Cyclohexenonderivate I bevorzugt, in denen $R^1$, $R^1$ und $R^2$ oder $R^1$ und $R^3$ die folgende Bedeutung haben:

$R^1$

$C_1$-$C_6$-Alkyl, insbesondere Methyl, Ethyl, n-Propyl, i-Propyl, n-Butyl, i-Butyl, s-Butyl, t-Butyl und n-Pentyl;
$C_2$-$C_6$-Alkenyl oder $C_2$-$C_6$-Alkinyl, insbesondere Vinyl, Propenyl, Ethinyl und Propinyl;
Cyclopropyl, Cyclobutyl, Cyclopentyl oder Cyclohexyl; Phenyl oder Pyridyl;

$R^2$

$C_1$-$C_4$-Alkyl, insbesondere Ethyl und n-Propyl;
partiell oder vollständig halogeniertes $C_1$-$C_3$-Alkyl, insbesondere 2-Fluorethyl und (E)-3-Chlorprop-2-en-1-yl;
$C_3$-$C_6$-Alkenyl, insbesondere Allyl und (E)-But-2-en-1-yl;
$C_3$-$C_6$-Alkinyl, insbesondere Propargyl und But-2-in-1-yl;
$C_1$-$C_4$-Alkyl, das einen 5- oder 6-gliedrigen aromatischen Heterocyclus mit einem Stickstoff, Sauerstoff oder Schwefelatom trägt, der seinerseits durch Halogen substituiert sein kann, insbesondere 5-Chlorthienylmethyl;
$C_3$-$C_6$-Alkenyl, das einen Phenyl- oder Halogenphenylrest trägt, insbesondere 4-Phenyl-but-2-en-1-yl, 4-(4-Fluorphenyl)-but-2-en-1-yl, 4-(4-Chlorphenyl)-but-2-en-1-yl, 4-(4-Fluorphenyl)-but-3-en-1-yl oder 4-(4-Chlorphenyl)-but-3-en-1-yl;
eine 3- bis 6-gliedrige Alkylkette, wobei ein Kettenglied durch Sauerstoff ersetzt ist, und wobei die Kette noch zusätzlich einen Phenyl- oder Halogenphenylrest tragen kann, insbesondere 2-(4-Chlorphenoxy)-ethyl, 2-(4-Fluorphenoxy)-ethyl, 2-(4-Chlorphenoxy)-propyl und 2-(4-Fluorphenoxy)-propyl;

$R^3$

Wasserstoff;
$C_1$-$C_6$-Alkyl, insbesondere Methyl, Ethyl, n-Propyl, i-Propyl, n-Butyl, s-Butyl, t-Butyl, n-Pentyl und n-Hexyl;
$C_3$-$C_6$-Alkenyl, insbesondere Allyl;
Hydroxy-$C_1$-$C_2$-alkyl, insbesondere Hydroxyethyl;
$C_1$-$C_2$-Alkoxy-$C_1$-$C_2$-alkyl, insbesondere 2-Methoxyethyl;
Phenyl oder Benzyl.

Ganz besonders bevorzugte Sübstituenten sind:

$R^1$ Methyl, Ethyl, n-Propyl, n-Butyl, Cyclopropyl und Phenyl;

$R^2$ Ethyl, Allyl, (E)-But-2-en-1-yl, Propargyl, But-2-in-1-yl, (E)-3-Chlorprop-2-en-1-yl, 4-(4-Fluorphenyl)-but-2-en-1-yl, 4-(4-Chlorphenyl)-but-2-en-1-yl, 4-(4-Fluorphenyl)-but-3-en-1-yl, 4-(4-Chlorphenyl)-but-3-en-1-yl, 2-(4-Chlorphenoxy)-propyl, 2-(4-Fluorphenoxy)-propyl und 5-Chlorethenyl;

$R^3$ Wasserstoff, Methyl, Ethyl, n-Propyl, Isopropyl, n-Butyl, sec.-Butyl, i-Butyl, n-Hexyl, Allyl, 2-Methoxyethyl, Benzyl, und Phenyl;

Als Salze der Verbindungen der Formel I kommen landwirtschaftlich brauchbare Salze, beispielsweise Alkalimetallsalze, insbesondere das Natrium- oder Kaliumsalz, Erdalkalisalze insbesondere das Calcium-, Magnesium- oder Bariumsalz, Mangan-, Kupfer-, Zink- oder Eisensalz sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Unter landwirtschaftlich brauchbaren Estern sind die Ester von

- $C_1$-$C_{10}$-Fettsäuren, insbesondere $C_1$-$C_6$-Alkylcarbonsäuren wie Methylcarbonsäure (Essigsäure), Ethylcarbonsäure (Propionsäure), Propylcarbonsäure (Buttersäure), 1-Methylethylcarbonsäure (Isobuttersäure), Butylcarbonsäure, 1-Methylpropylcarbonsäure, 2-Methylpropylcarbonsäure, 1,1-Dimethylethyl-Carbonsäure, Pentylcarbonsäure, 1-Methylbutylcarbonsäure, 2-Methylbutylcarbonsäure, 3-Methylbutylcarbonsäure, 1,1-Dimethylpropylcarbonsaure, 1,2-Dimethylpropylcarbonsäure, 2,2-Dimethylpropylcarbonsäure, 1-Ethylpropylcarbonsäure, Benzoesäure sowie durch Halogen substituierte Benzoesäuren, Hexylcarbonsäure, 1-Methylpentylcarbonsäure, 2-Methylpentylcarbonsäure, 3-Methylpentylcarbonsäure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethylbutylcarbonsäure, 1-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methylpropylcarbonsäure und 1-Ethyl-2-methylpropylcarbonsaure,
- $C_1$-$C_{10}$-Sulfonsäuren insbesondere $C_1$-$C_6$-Alkylsulfonsäuren wie Methylsulfonsäure, Ethylsulfonsäure, Propylsulfonsäure, 1-Methylethylsulfonsäure, Butylsulfonsäure, 1-Methylpropylsulfonsäure, 2-Methylpropylsulfonsäure, 1,1-Dimethylethylsulfonsäure, Pentylsulfonsäure, 1-Methylbutylsulfonsäure, 2-Methylbutylsulfonsäure, 3-Methylbutylsulfonsäure, 1,1-Dimethylpropylsulfonsäure, 1,2-Dimethylpropylsulfonsäure, 2,2-Dimethylpropylsulfonsäure, 1-Ethylpropylsulfonsäure, Benzolsulfonsäure sowie durch Halogen substituierte Benzolsulfonsäuren, Hexylsulfonsäure, 1-Methylpentylsulfonsäure, 2-Methylpentylsulfonsäure, 3-Methylpentylsulfonsäure, 4-Methylpentylsulfonsäure, 1,1-Dimethylbutylsulfonsäure, 1,2-Dimethylbutylsulfonsäure, 1,3-Dimethylbutylsulfonsäure, 2,2-Dimethylbutylsulfonsäure, 2,3-Dimethylbutylsulfonsäure, 3,3-Dimethylbutylsulfonsäure, 1-Ethylbutylsulfonsäure, 2-Ethylbutylsulfonsäure, 1,1,2-Trimethylpropylsulfonsäure, 1,2,2-Trimethylpropylsulfonsäure, 1-Ethyl-1-methylpropylsulfonsäure und 1-Ethyl-2-methylpropylsulfonsäure, und
- $C_1$-$C_{10}$-Phosphonsäuren, insbesondere $C_1$-$C_6$-Alkylphosphonsäuren wie Methylphosphonsäure, Ethylphosphonsäure, Propylphosphonsäure, 1-Methylethylphosphonsäure, Butylphosphonsäure, 1-Methylpropylphosphonsäure, 2-Methylpropylphosphonsäure, 1,1-Dimethylethylphosphonsäure, Pentylphosphonsäure, 1-Methylbutylphosphonsäure, 2-Methylbutylphosphonsäure, 3-Methylbutylphosphonsäure, 1,1-Dimethylpropylphosphonsäure, 1,2-Dimethylpropylphosphonsäure, 2,2-Dimethylpropylphosphonsäure, 1-Ethylpropylphosphonsäure, Benzolphosphonsäure sowie durch Halogen substituierte Benzolphosphonsäuren, Hexylphosphonsäure, 1-Methylpentylphosphonsäure, 2-Methylpentylphosphonsäure, 3-Methylpentylphosphonsäure, 4-Methylpentylphosphonsäure, 1,1-Dimethylbutylphosphonsäure, 1,2-Dimethylbutylphosphonsäure, 1,3-Dimethylbutylphosphonsäure, 2,2-Dimethylbutylphosphonsäure, 2,3-Dimethylbutylphosphonsäure, 3,3-Dimethylbutylphosphonsäure, 1-Ethylbutylphosphonsäure, 2-Ethylbutylphosphonsäure, 1,1,2-Trimethylpropylphosphonsäure, 1,2,2-Trimethylpropylphosphonsäure, 1-Ethyl-1-methylpropylphosphonsäure und 1-Ethyl-2-methylpropylphosphonsäure

zu verstehen.

Die Cyclohexenonderivate I sind auf verschiedene Weise erhältlich, und zwar vorzugsweise durch Umsetzung eines 5-substituierten Cyclohexan-1,3-dions der Formel II mit einem Säurederivat der Formel III:

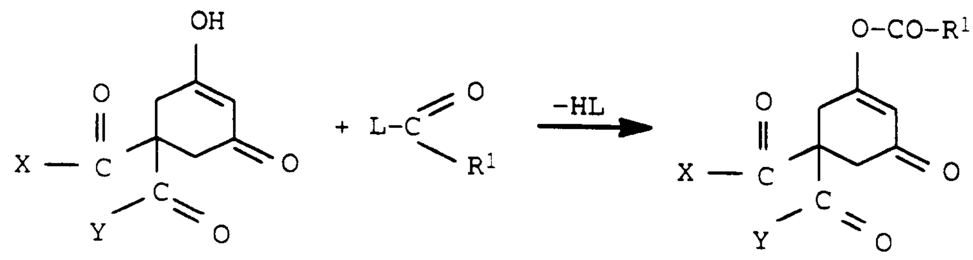


II III

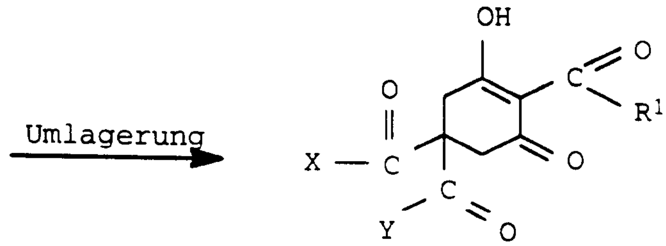


Ia

L bedeutet eine nukleophile Abgangsgruppe, insbesondere ein Halogenion wie Chlorid und Bromid.

Eine weitere Synthesemoglichkeit zur Herstellung der Cyclohexenonderivate Ia aus den Cyclohexan-1,3-dionen II wird in Tetrahedron Letters, 2491 (1975) beschrieben.

Die 5-substituierten Cyclohexenon-1,3-dione der Formel II lassen sich vorteilhaft in an sich bekannter Weise durch Cyclisierung von Verbindungen der allgemeinen Formel VI herstellen:

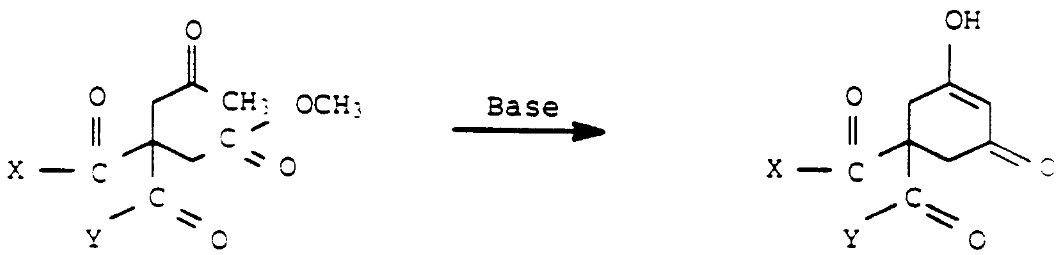


VI II

Die Cyclisierung erfolgt in Gegenwart einer starken Base, z.B. eines Alkalimetallhydrids wie Natriumhydrid, eines Lithiumorganyls wie Butyllithium oder eines Alkalimetallamids wie Lithiumdiisopropylamid und Natriumamid.

Zweckmäßig führt man die Umsetzung in homogener oder heterogener Phase in einem inerten Lösungs- oder Verdünnungsmittel durch.

Als Losungsmittel eignen sich beispielsweise Dimethylsulfoxid, Dimethylformamid, N-Methylpyrollidon, aromatische Kohlenwasserstoffe wie Benzol und Toluol, aliphatische Kohlenwasserstoffe wie Hexan und Cyclohexan oder Ether wie Dioxan und Tetrahydrofuran.

Im allgemeinen liegt die Reaktionstemperatur zwischen -100 °C und dem Siedepunkt des Reaktionsgemisches, vorzugsweise zwischen -70 °C und 40 °C.

Die Verbindungen der Formel VI lassen sich analog den in Izv. Akad. Nauk SSSR, Ser. Khim. 2, 473-474 (1990) beschrieben an Synthesen herstellen.

Cyclohexenonderivate der Formel Ib, können auf an sich bekannte Weise aus den Cyclohexenonderivaten Ia erhalten werden (vgl. DE-A-34 33 767):

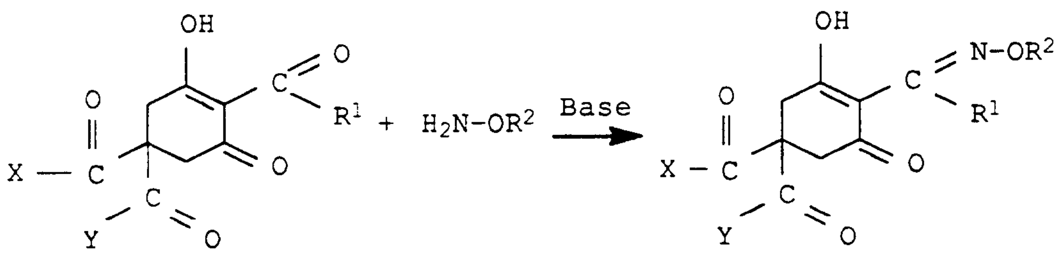


Ia Ib

Die Umsetzung kann sowohl mit dem Hydroxylamin $H_2N\text{-}OR_2$, bevorzugt in Form einer wäßrigen Lösung, als auch mit einem seiner Salze erfolgen.

Vorzugsweise verwendet man ein geeignetes Salz des Hydroxylamins $H_2N\text{-}OR^2$, insbesondere dessen Hydrochlorid, und führt die Reaktion in heterogener Phase in einem inerten Verdünnungsmittel durch, beispielsweise in Dimethylsulfoxid, in einem Alkohol wie Methanol, Ethanol und Isopropanol, in einem aromatischen Kohlenwasserstoff wie Benzol und Toluol, in einem chlorierten Kohlenwasserstoff wie Chloroform und 1,2-Dichlorethan, in einem aliphatischen Kohlenwasserstoff wie Hexan und Cyclohexan, in einem Ester wie Essigsäureethylester oder in einem Ether wie Dioxan und Tetrahydrofuran.

Bei der Reaktionsführung mit einer wäßrigen Lösung des Hydroxylamins $H_2N\text{-}OR^2$, erhält man je nach dem verwendeten Lösungsmittel für das Cyclohexenonderivat Ia ein ein- oder zweiphasiges Reaktionsgemisch.

Geeignete Lösungsmittel hierfür sind beispielsweise Alkohole wie Methanol, Ethanol und Isopropanol, aromatische Kohlenwasserstoffe wie Benzol und Toluol, chlorierte Kohlenwasserstoffe wie Chloroform und Dichlorethan, aliphatische Kohlenwasserstoffe wie Hexan und Cyclohexan, Ester wie Essigsäureethylester, Ether wie Dioxan und Tetrahydrofuran oder Nitrile wie Acetonitril.

Die Reaktionsführung erfolgt in Gegenwart einer Base, wobei z.B. die Carbonate, Hydrogencarbonate, Acetate, Alkoholate, Hydroxide oder Oxide von Alkali- und Erdalkalimetallen, vorzugsweise Natriumhydroxid, Kaliumhydroxid, Magnesiumoxid oder Calciumoxid, in Betracht kommen. Des weiteren sind organische Basen, z.B. tertiäre Amine wie Triethylamin und Pyridin, geeignet.

Normalerweise setzt man das Cyclohexenonderivat Ia und das Hydroxylamin $H_2N\text{-}OR^2$ bzw. dessen Salz in stöchiometrischem Verhältnis ein, jedoch kann in manchen Fällen auch ein Überschuß der einen oder anderen Komponente, bis etwa 10 mol-%, vorteilhaft sein.

Die Menge an Base ist nicht kritisch; in der Regel ist eine Menge zwischen 0,5 und 2 Mol, bezogen auf die Menge des Hydroxylamins $H_2N\text{-}OR^2$ ausreichend.

Die Reaktionstemperatur liegt im allgemeinen zwischen O °C und dem Siedepunkt des Reaktionsgemisches, vorzugsweise zwischen 20 und 80 °C.

Cyclohexantrione der Formel Ic sind auf an sich bekannte Weise durch Umsetzung von Cyclohexenonderivaten Ia mit Aminen $H_2N\text{-}R^3$ herstellbar:

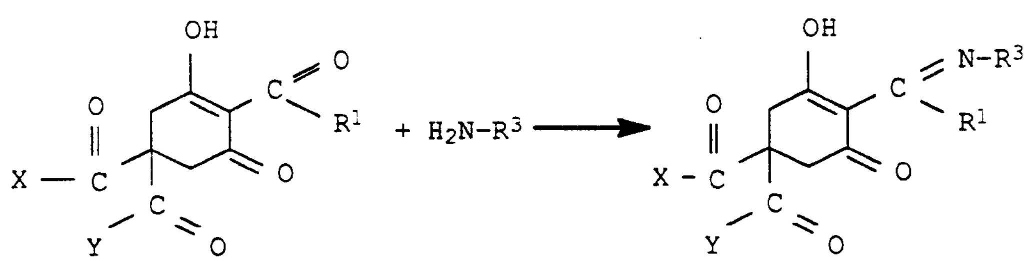

Ia Ic

In der Regel führt man die Umsetzung in homogener Phase in einem inerten Lösungsmittel durch.

Als Lösungsmittel eignen sich beispielsweise Dimethylsulfoxid, Alkohole wie Methanol, Ethanol und Isopropanol, aromatische Kohlenwasserstoffe wie Benzol und Toluol, chlorierte Kohlenwasserstoffe wie Chloroform und 1,2-Dichlorethan, aliphatische Kohlenwasserstoffe wie Hexan und Cyclohexan, Ester wie Essigsäureethylester oder Ether wie Dioxan und Tetrahydrofuran.

Zweckmäßigerweise setzt man die Ausgangsprodukte in ungefähr stochiometrischem Verhältnis ein, jedoch kann in manchen Fallen auch ein Überschuß der einen oder anderen Komponente, bis etwa 10 mol-%, vorteilhaft sein.

Im allgemeinen arbeitet man bei einer Reaktionstemperatur zwischen 0 °C und dem Siedepunkt des Reaktionsgemisches, vorzugsweise zwischen 20 und 80 °C.

Sämtliche vorstehend genannten Umsetzungen werden zweckmäßigerweise bei Normaldruck vorgenommen. Geringerer oder höherer Druck ist möglich, bringt im allgemeinen aber keine Vorteile.

Die Aufarbeitung der jeweiligen Reaktionsgemische auf die Produkte hin erfolgt mittels üblicher Ausarbeitungsmethoden, vorzugsweise durch Einengen der Mischung, Verteilung des Rückstandes in Methylenchlorid/Wasser und Abdestillieren des Lösungsmittels unter vermindertem Druck.

Die erfindungsgemäßen Cyclohexenonderivate können Salze von Alkali- oder Erdalkalimetallverbindungen sowie Enolester bilden.

Durch Behandeln der Verbindungen Ia, Ib oder Ic mit Natrium- oder Kaliumhydroxid oder -alkoholat in wäßriger Lösung oder in einem organischen Lösungsmittel, z.B. einem Alkohol wie Methanol und Ethanol, einem aromatischen Kohlenwasserstoff wie Toluol oder einem aprotischen Lösungsmittel wie Aceton, können Alkalimetallsalze der Cyclohexenonderivate I hergestellt werden.

Andere Metallsalze, z. B. die Mangan-, Kupfer-, Zink-, Eisen-, Calcium-, Magnesium- und Bariumsalze, können aus den Natriumsalzen der Cyclohexenonderivate Ia bzw. Ib in üblicher Weise hergestellt werden, ebenso Ammonium-, Phosphonium-, Sulfonium oder Sulfoxoniumsalze mittels Ammoniak, Phosphonium-, Sulfonium- oder Sulfoxoniumhydroxiden.

Die Cyclohexenonderivate I können bei der Herstellung als Isomerengemische anfallen, wobei sowohl E-/Z-Isomerengemische als auch Enantiomeren- oder Diastereomerengemische möglich sind. Die Isomerengemische können gewünschtenfalls nach den hierfür üblichen Methoden, z.B. durch Chromatographie oder durch Kristallisation, getrennt werden.

Die Cyclohexenonderivate I können in mehreren tautomeren Formen geschrieben werden, die alle von der Erfindung umfaßt werden.

Die Cyclohexenonderivate Ib eignen Sich, sowohl als Isomerengemische als auch in Form der reinen Isomeren, als Herbizide, insbesondere zur Bekämpfung von Pflanzenarten aus der Familie der Gramineen (Gräser). Im allgemeinen sind sie verträglich und somit selektiv in breitblättrigen Kulturen sowie in Monokotylen (einkeimblättrigen Gewächsen).

In Abhängigkeit von der jeweiligen Applikationsmethode können die Cyclohexenonderivate Ib bzw. die sie enthaltenden Mittel in einer Vielzahl von Kulturpflanzen zur Beseitigung unerwünschter Pflanzen eingesetzt werden, wobei die folgenden Kulturen beispielhaft genannt seien:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |

| Botanischer Name | Deutscher Name |
|---|---|
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Phaseolus lunatus | Mondbohne |
| Phaseolus vulgaris | Buschbohnen |
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |

| Botanischer Name | Deutscher Name |
|---|---|
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (S. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Die Cyclohexenonverbindungen der Formel Ia und Ic können die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig

a) von der Pflanzenart und -sorte,

b) von dem Zeitpunkt der Applikation, bezogen auf das Entwicklungsstadium der Pflanze, und von der Jahreszeit,

c) von dem Applikationsort und -verfahren (z.B. Samenbeize, Bodenbehandlung, Blattapplikation oder Stamminjektion bei Bäumen),

d) von klimatischen Faktoren (z.B. Temperatur, Niederschlagsmenge, außerdem auch Tageslänge und Lichtintensität),

e) von der Bodenbeschaffenheit (einschließlich Düngung),

f) von der Formulierung bzw. Anwendungsform des Wirkstoffs und

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:

A. Mit den erfindungsgemäß verwendbaren Verbindungen läßt sich das vegetative Wachstum der Pflanzen stark hemmen, was sich insbesondere in einer Reduzierung des Langenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Ein-setzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird,
- eine dickere Epidermis und Cuticula ausgebildet werden,
- die Durchwurzelung des Bodens verbessert wird und
- das Mikroklima im Pflanzenbestand durch einen kompakteren Wuchs günstig beeinflußt wird.

Besonders gut eignen sich die Cyclohexenonverbindungen Ia und Ic zur Halmverkürzung von Kulturpflanzen wie Gerste, Raps und Weizen.

Die erfindungsgemäß zu verwendenden Wachstumsregulatoren der Formel Ia und Ic können den Kulturpflanzen sowohl vom Samen her (als Saatgutbeizmittel) als auch über den Boden, d.h. durch die Wurzel sowie - besonders bevorzugt - durch Spritzung über das Blatt, zugeführt werden.

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Die Wirkstoffe Ia, Ib und Ic können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als inerte Hilfsstoffe kommen dafür im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, Lösungsmittel wie Aromaten (z.B. Toluol, Xylol), chlorierte Aromaten (z.B. Chlorbenzole), Paraffine (z.B. Erdölfraktionen), Alkohole (z.B. Methanol, Ethanol, Butanol, Cyclohexanol), Ketone (z.B. Cyclohexanon, Isophoron), Amine (z.B. Ethanolamin, N,N-Dimethylformamid, N-Methylpyrrolidon) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Ligninsulfitablaugen oder Methylcellulose in Betracht.

Pulver-, Streu- und Stäubemittel können durch Mischen oder gemeinsames Vermahlen der wirksamen Substanzen mit einem festen Tragerstoff hergestellt werden.

Granulate, z.B. Umhüllungs-, Imprägnierungs- und Homogengranulate konnen durch Bindung der Wirkstoffe an feste Tragerstoffe hergestellt werden. Feste Tragerstoffe sind Mineralerden wie Kieselsauren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Dungemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Tragerstoffe.

Die Konzentrationen der Wirkstoffe Ia, Ib und Ic in den anwendungsfertigen Zubereitungen können in weiten Bereichen variiert werden, etwa zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen der Verbindung Nr. 1.1 und 10 Gew.-Teilen N-Methyl-a-pyrrolidon, die zur Anwendung in Form kleinster Tropfen geeignet ist;

II. eine Mischung aus 20 Gew.-Teilen der Verbindung Nr. 1.2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calcium-salz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.

III. eine wäßrige Dispersion aus 20 Gew.-Teilen der Verbindung Nr. 1.1, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.

IV. eine wäßrige Dispersion aus 20 Gew.-Teilen der Verbindung Nr. 1.2, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;

V. eine in einer Hammermühle vermahlene Mischung aus 80 Gew.-Teilen der Verbindung Nr. 1.1, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel. Durch

feines Verteilen der Mischung in 20 000 Gew.-Teilen Wasser erhält man eine Spritzbrühe, die 0,1 Gew.-% des Wirkstoffs enthält;

VI. eine innige Mischung aus 3 Gew.-Teilen der Verbindung Nr. 1.2 und 97 Gew.-Teilen feinteiligem Kaolin. Dieses Stäubemittel enthält 3 Gew.-% Wirkstoff;

VII. eine innige Mischung aus 30 Gew.-Teilen der Verbindung Nr. 1.1, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;

VIII. eine stabile wäßrige Dispersion aus 40 Gew.-Teilen der Verbindung Nr. 1.2, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 1.1, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-harnstoff-formaldehyd-Kondensates und 68 Gew.-Teilen eines paraffinischen Mineralöls;

X. eine in einer Hammermühle vermahlene Mischung aus 10 Gew.-Teilen der Verbindung Nr. 1.2, 4 Gew.-Teilen des Natriumsalzes der Diisobutylnaphthalin-a-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Applikation der Wirkstoffe bzw. der herbiziden und das Pflanzenwachstum regulierenden Mittel kann im Vorauflauf- oder im Nachauflaufverfahren erfolgen. Normalerweise werden die Pflanzen mit den Wirkstoffen besprüht oder bestäubt oder die Samen der Testpflanzen mit den Wirkstoffen behandelt. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 2 kg/ha aktive Substanz (a.S.).

Zur Verbreiterung des Wirkungsspektrums und zur Erzielung synergistischer Effekte können die Cyclohexenonderivate I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Die Cyclohexenonderivate Ia, Ib und Ic können auch gemeinsam mit anderen Pflanzenschutzmitteln wie Herbiziden, Wachstumsregulatoren, Schädlingsbekämpfungsmittel, Fungiziden und Bakteriziden ausgebracht werden. Diese Mittel können zu den erfindungsgemäßen Mitteln im Gewichtsverhältnis 1:100 bis 100:1 zugemischt werden, gewünschtenfalls auch erst unmittelbar vor der Anwendung (Tankmix). Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Herstellungsbeispiele

### Beispiel 1

4-Cyclopropylcarbonyl-3,5-dioxo-cyclohexan-1,1-dicarbonsäurediethylester (Verbindung Nr. 1.1)

Zu einer Lösung von 1,2 g 3-Cyclopropylcarbonyloxy-5-oxo-cyclohex-3-en-1,1-dicarbonsäurediethylester in 20 ml Dichlormethan wurden 0,1 g Dimethylaminopyridin gegeben, wonach man das Reaktionsgemisch 4 Tage bei 20-25 °C rührte. Anschließend entfernte man das Lösungsmittel unter reduziertem Druck und reinigte den Rückstand chromatographisch (Laufmittel: Cyclohexan/Essigester 8:2 bis 1:2). Ausbeute: 0,6 g.

Vorstufe 1a)

2-Carbethoxy-4-oxo-valeriansäureethylester

Zu einer Mischung aus 9 g (0,162 mol) feingepulvertem Kaliumhydroxid, 24,7 ml (0,162 mol) Diethylmalonat, ca. 0,2 g Benzyltriethylammoniumchlorid und 150 ml Dimethylformamid wurden unter schnellem Rühren 15 g (0,162 mol) Chloraceton getropft. Nach Beendigung der exotherme Reaktion rührte man die Mischung noch 2 bis 3 Stunden bei 45 °C und ließ sie dann auf 20-25 °C abkühlen. Anschließend verteilte man die Reaktionsprodukte zwischen Essigsäureethylester und Wasser, wonach die organische Phase dreimal mit je 100 ml Wasser gewaschen, über Natriumsulfat getrocknet und eingeengt wurde. Ausbeute: 30.6 g eines Öls das nach gaschromatographischer Analyse zu ca. 61 % das Produkt enthält. Die Reinigung erfolgt durch fraktionierte Destillation($Kp_{0,1\ Torr}$: 87 - 88 °C).

$^1$H-NMR (in $CDCl_3$; TMS als interner Standard): $\delta$ = 1,3 ppm (t,6H); 2,2 ppm (s,3H); 3,08 ppm (d,2H); 3,88 ppm (t,1H); 4,22 ppm (q,4H).

Vorstufe 1b)

3,3-Biscarbethoxy-5-oxo-hexancarbonsäureethylester

Zu einer Suspension von 2,1 g (0,037 mol) feingepulvertem Kaliumhydroxid, 8 g (0,037 mol) 2-Carbethoxy-4-oxo-valeriansäureethylester, ca. 0,1 g Benzyltriethylammoniumchlorid und 150 ml Dimethylformamid tropfte man 4 ml (0,037 mol) Chloressigsäureethylester. Nach etwa 15 Stunden Rühren bei 20-25 °C wurden zur Vervollständigung der Umsetzung nochmals 2 ml (0,017 mol) Chloressigsäureethylester und 1 g (0,016 mol) Kaliumhydroxid zugegeben. Man rührte nochmals 2 Stunden bei 20-25 °C, wonach man die Reaktionsprodukte zwischen Essigsäureethylester und 20 gew.-%iger Ammoniumchloridlosung verteilte. Die organische Phase wurde zweimal mit je 50 ml 20 gew.-%iger Ammoniumchloridlösung gewaschen, danach getrocknet und eingeengt. Ausbeute: 9,5 g eines Öls, das 91 % des Produktes enthielt. Die Reinigung erfolgt durch fraktionierte Destillation ($Kp_{0,1\ Torr}$: 135 °C).

$^1$H-NMR (in $CDCl_3$; TMS als interner Standard): 1,25 ppm (t,9H); 2,15 ppm (s,3H); 3,13 ppm (s,2H); 3,35 ppm (s,2H); 4,05-4.3 ppm (m,6H).

Vorstufe 1c)

5,5-Biscarbethoxy-cyclohexan-1,3-dion

Zu einer Suspension aus 3,6 g (0,118 mol) 80 gew-%iges Natriumhydrid in 200 ml Dimethylformamid wurde eine Losung von 14,3 g (0,047 mol) 3,3-Biscarbethoxy-5-oxo-hexancarbonsäureethylester in 40 ml Dimethylformamid getropft. Nach ca. 15 Stunden Rühren bei 20-25 °C wurde die Reaktion durch Zugabe von 5 ml Isopropanol abgebrochen. Man verteilte die Reaktionsprodukte zwischen Essigester und 1-molarer Salzsaure, wonach die organische Phase abgetrennt, getrocknet und eingeengt wurde. Ausbeute: 2,2 g eines Rohproduktöls.

$^1$H-NMR (in $CDCl_3$; TMS als interner Standard): $\delta$ = 1,28 ppm (t,6H); 2,98 ppm (s,4H); 4,25 ppm (q,4H); 5,5 ppm (s,1H); 9,2 ppm (bs,1H).

Vorstufe 1d)

3-Cyclopropylcarbonyloxy-5-oxo-cyclohex-3-en-1,1-dicarbonsaurediethylester

Eine Lösung von 2,4 g (9,4 mmol) 5,5-Biscarbethoxy-cyclohexan-1,3-dion in 25 ml trockenem Tetrahydrofuran wurde mit 0,94 g (9,4 mmol) Triethylamin versetzt. Zu diesem Gemisch tropfte man bei 0 bis 10 °C eine Lösung von 0,98 g (9,4 mmol) Cyclopropancarbonsäurechlorid in 5 ml Tetrahydrofuran. Nach etwa einer Stunde Rühren bei 0 bis 10 °C konnte dunnschichtchromatographisch kein Ausgangsmaterial mehr festgestellt werden, wonach man das Lösungsmittel entfernte und den Rückstand in Essigester aufnahm. Die organische Phase wurde zweimal mit 1 %iger Essigsäure und zweimal mit Wasser gewaschen, getrocknet und eingeengt. Das Rohprodukt wurde chromatographisch (Laufmittel: Cyclohexan/Essigester 8:2) gereinigt. Ausbeute: 1,2 g.

In der folgenden Tabelle 1 sind noch weitere Verbindungen I aufgeführt, die auf die gleiche weise hergestellt wurden oder herstellbar sind.

Tabelle 1

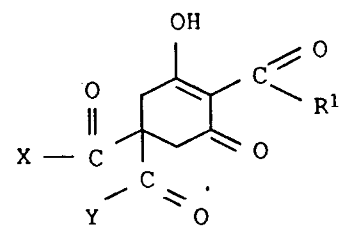

Ia

| Nr. | $R^1$ | X | Y | $^1$H-NMR (δ [ppm], in $CDCl_3$) |
|---|---|---|---|---|
| 1.1 | Cyclopropyl | O-Ethyl | O-Ethyl | 1,15 (m, 2H); 1,27 (t, 3H); 1,3 (m, 2H); 3,0 (s,2H); 3,18 (s, 2H); 3,55 (m, 1H); 4,25 (q, 4H); |
| 1.2 | Ethyl | O-Ethyl | O-Ethyl | 1,1 (t, 3H); 1,25 (t, 6H); 2,96 (s, 2H); 3,08 (q, 2H); 3,18 (s, 2H); 4,25 (q, 4H); |
| 1.3 | Ethyl | O-Methyl | O-Methyl | 1,14 (t,3H); 2,99 (s,2H); 3,07 (q,2H); 3,21 (s,2H); 3,78 (s,6H); |
| 1.4 | Methyl | O-Methyl | O-Methyl | 2,59 (s,3H), 2,98 (s,2H); 3,2 (s,2H); 3,75 (s,6H); |
| 1.5 | Cyclopropyl | O-Methyl | O-Methyl | 1,17 (m,2H); 1,33 (m,2H); 3,02 (s,2H); 3,18 (s,2H); 3,55 (m,1H); 3,77 (s,6H); |
| 1.6 | Propyl | O-Methyl | O-Methyl | 0,97 (t,3H); 1,62 (m,2H); 2,97 (m,4H); 3,2 (s,2H); 3,76 (s,6H); |

| Nr. | $R^1$ | X | Y | $^1H$-NMR (δ [ppm], in $CDCl_3$) |
|---|---|---|---|---|
| 1.7 | 4-Chlorphenyl | O-Methyl | O-Methyl | 3,13 (bs,4H); 3,83 (s,6H); 7,4 (d,2H); 7,48 (d,2H); |
| 1.8 | 4-Chlor-2-nitrophenyl | O-Methyl | O-Methyl | 2,84 (s,2H); 3,33 (s,2H); 3,79 (s,6H), 7,21 (d,1H); 7,68 (dd,1H); 8,19 (d,1H); |
| 1.9 | 4-Chlorphenyl | OH | O-Methyl | 3,0(bs,4H); 3,74 (s,3H); 7,55 (d,3H); 7,69 (d,2H); |

Tabelle 2

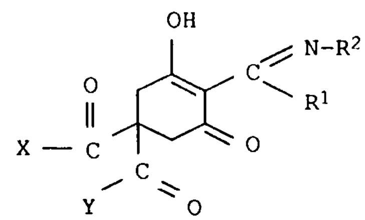

mit X und Y = O-Methyl

| Nr. | $R^1$ | $R^2$ | $^1H$-NMR |
|---|---|---|---|
| 2.1 | Methyl | i-Propyl | 1,29 (d,6H); 2,56 (s,3H); 2,99 (s,4H); 3,73 (s,6H); 3,97 (m,1H); |
| 2.2 | Methyl | i-Butyl | 1,09 (d,6H); 1,96 (m,1H); 2,52 (s,3H); 2,98 (s,4H); 3,22 (t,2H); 3,74 (s,6H); |
| 2.3 | Methyl | s-Butyl | 0,96 (t,3H); 1,26 (d,3H); 1,65 (m,2H); 2,53 (s,3H); 3,0 (s,4H); 3,73 (m,7H); |
| 2.4 | Ethyl | i-Propyl | 1,19 (s,3H); 1,3 (d,6H); 2,99 (s,4H); 3,02 (q,2H); 3,75 (s,6H); 4,06 (m,1H); |
| 2.5 | Ethyl | s-Butyl | 0,95 (t,3H); 1,17 (t,3H); 1,28 (d,3H); 1,64 (m,2H); 2,99 (m,6H); 3,72 (s,6H); |
| 2.6 | Ethyl | i-Butyl | 1,04 (d,6H); 1,17 (t,3H); 1,98 (m,1H); 3,0 (m, 6H); 3,25 (m,2H); 3,75 (s,6H); |
| 2.7 | Propyl | i-Propyl | 1,04 (t,3H); 1,29 (d,6H); 1,52 (m,2H); 2,96 (m,6H); 3,75 (s,6H); 3,95 (m,1H); |

| Nr. | $R^1$ | $R^2$ | $^1H$-NMR |
|---|---|---|---|
| 2.8 | Propyl | s-Butyl | 0,94 (m,3H); 1,05 (t,3H); 1,28 (d,3H); 1,62 (m,4H); 2,97 (m,6H); 3,72 (m,7H); |
| 2.9 | Propyl | i-Butyl | 0,94 (m,3H); 1,05 (d,6H); 1,54 (m,2H); 1,96 (m,1H); 2,94 (m,2H); 2,97 (s,4H); 3,25 (m,2H); 3,73 (s,6H); |
| 2.10 | Methyl | Benzyl | 2,69 (s,3H); 2,99 (s,4H); 3,73 (s,6H); 4,58 (d,2H); 7,27 (m,5H) |

Tabelle 3

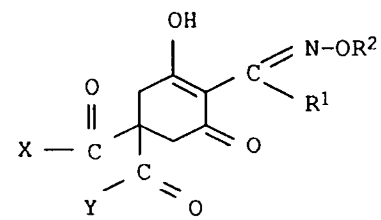

mit X = Y = O-Methyl

| Nr. | $R^1$ | $R^2$ | $^1$H-NMR |
|---|---|---|---|
| 3.1 | Methyl | Ethyl | 1,32 (s,3H); 2,39 (s,3H); 3,02 (s,4H); 3,78 (s,6H); 4,11 (q,2H); |
| 3.2 | Methyl | Allyl | 2,38 (s,3H); 2,92 (bs,2H); 3,13 (bs,2H); 3,74 (s,6H); 4,52 (d,2H); 5,38 (m,2H); 5,98 (m,1H); |
| 3.3 | Methyl | t-Chlorallyl | 2,34 (s,3H); 3,03 (bs,4H); 3,74 (s,6H); 4,54 (d,2H); 6,11 (m,1H); 6,32 (d,1H); |
| 3.4 | Methyl | 2-(4-Chlorphenoxy)propyl | 1,34 (d,3H); 2,29 (s,3H); 2,9 (bs,2H); 3,14 (bs,2H); 3,75 (s,6H); 4,19 (m,2H); 4,59 (m,1H); 6,87 (d,2H); 7,20 (d,2H); |
| 3.5 | Ethyl | Ethyl | 1,1 (t,3H); 1,34 (t,3H); 2,97 (q,2H); 3,04 (m,4H); 3,75 (s,6H); 4,1 (q,2H); |
| 3.6 | Ethyl | Allyl | 1,09 (t,3H); 2,9 (q,2H), 3,78 (s,6H); 4,54 (d,2H); 5,35 (m,2H); 5,92 (m,1H); |
| 3.7 | Ethyl | t-Chlorallyl | 1,07 (t,3H); 2,86 (q,2H); 3,74 (s,6H); 4,5 (d,2H); 6,12 (m,1H); 6,34 (d,1H); |

| Nr. | $R^1$ | $R^2$ | $^1$H-NMR |
|---|---|---|---|
| 3.8 | Ethyl | 2-(4-Chlorphenoxy)propyl | 1,04 (t,3H); 1,33 (d,3H); 2,82 (q,2H); 2,94 (m,2H); 3,16 (m,2H); 3,78 (s,6H); 4,2 (m,2H); 4,6 (m,1H); 6,88 (d,2H); 7,2 (d,2H); |
| 3.9 | Propyl | Ethyl | 0,95 (t,3H); 1,34 (t,3H); 1,5 (m,2H); 2,94 (m,2H); 3,04 (s,4H); 3,74 (s,6H); 4,13 (q,2H); |
| 3.10 | Propyl | Alkyl | 0,94 (t,3H); 1,5 (m,2H); 2,93 (m,2H); 3,04 (bs,4H); 3,79 (s,6H); 4,55 (d,2H); 5,38 (m,2H); 5,97 (m,1H); |
| 3.11 | Propyl | t-Chloralkyl | 0,92 (t,3H); 1,46 (m,2H); 2,9 (m,4H); 3,15 (m,2H); 3,76 (s,6H); 4,52 (d,2H); 6,09 (m,1H); 6,33 (d,1H); |
| 3.12 | Propyl | 2-(4-Chlorphenoxy)propyl | 0,87 (t,3H); 1,31 (d,3H); 1,45 (m,2H); 2,9 (m,4H); 3,14 (m,2H); 3,76 (s,6H); 4,19 (m,2H); 4,60 (m,1H); 6,83 (d,2H); 7,2 (d,2H); |

Anwendungsbeispiele

Die herbizide und die das Pflanzenwachstum regulierende Wirkung der substituierten Cyclohexenone I, Ia und Ib ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen bereits in den Versuchsgefäßen angezogen oder einige Tage vorher in die Versuchsgefäße verpflanzt. Die Applikation der in Wasser suspendierten oder emulgierten Wirkstoffe erfolgte je nach Wuchsform erst bei einer Wuchshöhe von 3 bis 15 cm.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Abkürzung | Lateinischer Name | Deutscher Name |
|---|---|---|
| ECHCG | Echinochloa crus-galli | Hühnerhirse |
| BROSS | Bromus spp. | Trespenarten |
| SETIT | Setaria italica | Kolbenhirse |

Mit 3 kg/ha aktive Substanz im Nachauflaufverfahren eingesetzt lassen sich die unerwünschten Pflanzen mit den Beispielen 3.6 und 3.7 sehr gut bekämpfen.

Die wachstumsregulierende Wirkung der Verbindungen der allgemeinen Formel I ließ sich durch folgende Versuche zeigen:

Die Wirkstoffe wurden entweder

a) als 0,1 gew.-%ige Lösung in Aceton oder

b) als 10 gew.-%ige Emulsion in einem Gemisch aus 70 Gew.-% Cyclohexanol, 20 Gew.-% Nekanil® LN (Lutensol® AP6; Netzmittel mit Emulgier- und Dispergierwirkung auf der Basis ethoxylierter Alkylphenole) und 10 Gew.-% Emulphor® EL (Emulan® EL; Emulgator auf der Basis ethoxylierter Fettalkohole)

aufgearbeitet und entsprechend der gewünschten Konzentration mit Aceton (Fall a)) oder mit Wasser (Fall b)) verdünnt.

Bei Versuchsende wurden die Wuchshöhen der behandelten Pflanzen gemessen und zur Wuchshöhe von nicht behandelten Pflanzen in Relation gesetzt. Als Vergleichssubstanz zur Beurteilung der wachstumsregulierenden Wirkung diente N-(2-Chlorethyl)-N,N,N-trimethyl-amoniumchlorid (Vergleichsverbindung A).

Die nachfolgenden Tabellen zeigen beispielhaft die Halmverkürzung von Raps-, Gerste- und Weizenpflanzen, die mit den Cyclohexenonderivaten 1.1 bis 1.6 und 2.1, 2.3, 2.5 bis 2.8 behandelt wurden:

| Verb. Nr. | Aufwandmenge [kg/ha a.S.] | Testpflanzen und relative Wuchshöhe*) | | |
|---|---|---|---|---|
| | | Winterraps Sorte "Librador" | Sommergerste Sorte "Alexis" | Sommerweizen Sorte "Star" |
| 1.1 | 0,75 | 74 | 74 | 92 |
| 1.2 | 0,75 | 87 | 74 | 92 |
| A | 0,75 | 94 | 106 | 92 |
| 1.1 | 0,38 | 74 | 88 | 92 |
| 1.2 | 0,38 | 94 | 92 | 98 |
| A | 0,38 | 101 | 106 | 92 |

*) 100 = kein Effekt

| Verb. Nr. | Aufwandmenge [kg/ha a.S.] | Testpflanzen und relative Wuchshöhe*) | | |
|---|---|---|---|---|
| | | Winterraps Sorte "Librador" | Sommergerste Sorte "Alexis" | Sommerweizen Sorte "Star" |
| CCC | 3 | 75 | 92 | 97 |
| 1.3 | 1,5 | 75 | 68 | 86 |
| 1.4 | 1,5 | 69 | 82 | 93 |
| 1.5 | 1,5 | 56 | 71 | 86 |
| 1.6 | 1,5 | 69 | 78 | 90 |
| 2.1 | 1,5 | 75 | 78 | 97 |
| 2.3 | 1.5 | 81 | 82 | 97 |
| 2.5 | 1,5 | 88 | 75 | 86 |
| 2.6 | 1,5 | 88 | 68 | 86 |
| 2.7 | 1,5 | 81 | 92 | 93 |
| 2.8 | 1,5 | 81 | 92 | 100 |

*) 100 = kein Effekt

**Patentansprüche**
**Patentansprüche für folgende Vertragsstaaten : AT, BE, CH, LI, DE, FR, GB, IT, NL**

**1.** Cyclohexenonderivate der allgemeinen Formel I

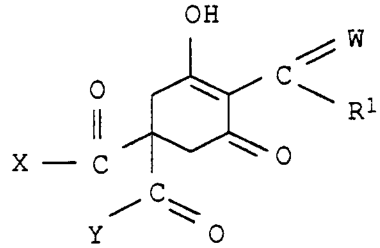

I

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy, $C_1$-$C_4$-Alkylsulfinyl und $C_1$-$C_4$-Alkylsulfonyl;

W Sauerstoff, eine Gruppe =N-O$R^2$ oder =N-$R^3$, mit

$R^2$

- einen $C_1$-$C_6$-Alkyl-, $C_3$-$C_6$-Alkenyl- oder $C_3$-$C_6$-Alkinylrest, wobei diese Reste noch einen bis drei der folgen den Substituenten tragen können, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;
- eine 3- bis 6-gliedrige Alkylkette oder eine 4- bis 6-gliedrige Alkenyl- oder Alkinylkette, wobei jeweils eine Kettenglied durch Sauerstoff, Schwefel oder eine Gruppe -SO-, -$SO_2$- oder -N($R^8$)- ersetzt ist, und wobei die Kette noch zusätzlich ein bis drei Substituenten tragen kann, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;

$R^8$ steht für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl;

$R^3$

Wasserstoff, einen $C_1$-$C_6$-Alkylrest, einen Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, den Phenyl- oder Benzylrest, wobei die aromatischen Ringe noch ein bis drei Substituenten tragen können, ausgewählt aus einer Gruppe bestehend aus Nitro, Halogen, $C_1$-$C_4$-Alkyl,$C_1$-$C_4$-Halogenalkyl und $C_1$-$C_4$-Alkoxy;

X,Y eine Gruppe -O$R^4$ oder -N$R^5R^6$,

$R^4$

Wasserstoff, einen $C_1$-$C_6$-Alkyl-, $C_3$-$C_6$-Alkenyl-, $C_3$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_2$-alkyl-, $C_1$-$C_4$-Alkylthio-$C_1$-$C_2$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe -N($R^7$)- als Ringglied enthalten kann, wobei

$R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht,

sowie die landwirtschaftlich brauchbaren Salze von I und die Ester von I mit $C_1$-$C_{10}$-Carbonsäuren oder anorganischen Säuren.

**2.** Verfahren zur Herstellung von Cyclohexenonderivaten der Formel Ia

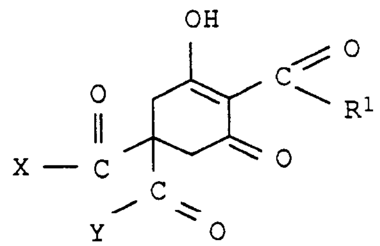

Ia

wobei die Variablen die folgende Bedeutung haben:

- $R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl $C_1$-$C_4$-Alkoxy, $C_1$-$C_4$-Alkylsulfinyl und $C_1$-$C_4$-Alkylsulfonyl;
- X,Y eine Gruppe $-OR^4$ oder $-NR^5R^6$, wobei
- $R^4$ Wasserstoff, einen $C_1$-$C_6$-Alkyl-, $C_2$-$C_6$-Alkenyl-, $C_2$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,
- $R^5$ Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl, $C_1$-$C_4$-Alkoxy-$C_1$-$C_4$-alkyl- oder $C_1$-$C_4$-Alkylthio-$C_1$-$C_4$-alkylrest, und
- $R^6$ Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, partiell oder vollständig halogeniertem $C_1$-$C_4$-Halogenalkyl, $C_1$-$C_4$-Alkoxy und $C_1$-$C_4$-Alkylthio; oder
  $R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe $-N(R^7)$- als Ringglied enthalten kann, wobei
- $R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht, bedeuten,

dadurch gekennzeichnet, daß man ein 5-substituiertes Cyclohexan-1,3-dion der allgemeinen Formel II

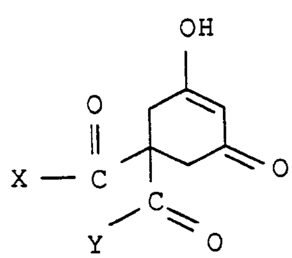

II

mit einem Säurederivat der allgemeinen Formel III,

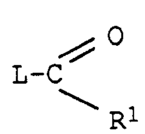

III

in der L für eine nukleophile Abgangsgruppe steht, umsetzt.

3. Verfahren zur Herstellung von Cyclohexenonderivaten der Formel

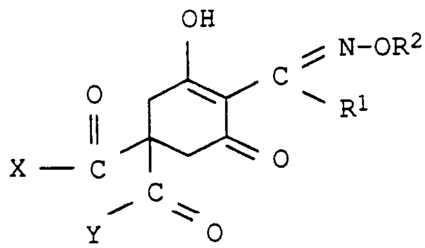

Ib

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl $C_1$-$C_4$-Alkoxy, $C_1$-$C_4$-Alkylsulfinyl und $C_1$-$C_4$-Alkylsulfonyl;

$R^2$ einen $C_1$-$C_6$-Alkyl-, $C_3$-$C_6$-Alkenyl- oder $C_3$-$C_6$-Alkinylrest, wobei diese Reste noch einen bis drei der folgenden Substituenten tragen können, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl und $C_1$-$C_4$-Alkoxy; eine 3- bis 6-gliedrige Alkylkette oder eine 4- bis 6-gliedrige Alkenyl- oder Alkinylkette, wobei jeweils eine Kettenglied durch Sauerstoff, Schwefel oder eine Gruppe -SO-, $-SO_2-$ oder $-N(R^8)-$ ersetzt ist, und wobei die Kette noch zusätzlich ein bis drei Substituenten tragen kann, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;

$R^8$ steht für Wasserstoff, $C_1$-$C_6$-Alkyl, $C_1$-$C_6$-Alkenyl, $C_1$-$C_6$-Alkinyl, $C_1$-$C_6$-Alkylcarbonyl oder Benzoyl;

X,Y eine Gruppe $-OR^4$ oder $-NR^5R^6$, wobei

$R^4$

Wasserstoff, einen $C_1$-$C_6$-Alkyl-, $C_2$-$C_6$-Alkenyl-, $C_2$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$

Wasserstoff, einen $C_1$-$C_6$-Alkyl-, $C_2$-$C_6$-Alkenyl-, $C_2$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$

Wasserstoff, einen $C_1$-$C_6$-Alkyl-, $C_2$-$C_6$-Alkenyl-, $C_2$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe $-N(R^7)-$ als Ringglied enthalten kann, wobei

$R^7$ für Wasserstoff, $C_1$-$C_6$-Alkyl, $C_1$-$C_6$-Alkenyl,
$C_1$-$C_6$-Alkinyl, $C_1$-$C_6$-Alkylcarbonyl oder Benzoyl steht,

bedeuten,

dadurch gekennzeichnet, daß man eine Verbindung der Formel Ia

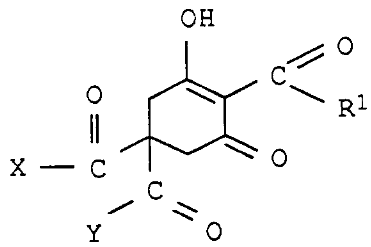

Ia

mit einem Hydroxylamin der allgemeinen Formel IV

$H_2N\text{-}O\text{-}R^2$ IV

umsetzt.

**4.** Verfahren zur Herstellung von Cyclohexenonderivaten der Formel

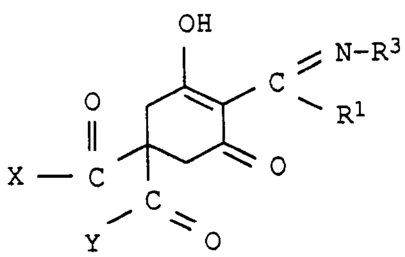

Ic

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein-bis drei Reste tragen können, ausgewählt aus einer Gruppe bestehend aus Nitro, Cyano, Halogen, $C_1$-$C_4$-Alkyl, $C_1$-$C_4$-Halogenalkyl $C_1$-$C_4$-Alkoxy, $C_1$-$C_4$-Alkylsulfinyl und $C_1$-$C_4$-Alkylsulfonyl;

$R^3$ Wasserstoff, einen $C_1$-$C_6$-Alkylrest, einen Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, den Phenyl- oder Benzylrest, wobei die aromatischen Ringe noch ein bis drei Substituenten tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;

X,Y eine Gruppe $-OR^4$ oder $-NR^5R^6$, wobei

$R^4$

Wasserstoff, einen $C_1$-$C_6$-Alkyl-, $C_3$-$C_6$-Alkenyl-, $C_3$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest,oder den Benzoylrest, der noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe $-N(R^7)-$ als Ringglied enthalten kann, wobei

$R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht,

bedeuten,

dadurch gekennzeichnet, daß man eine Verbindung der Formel Ia

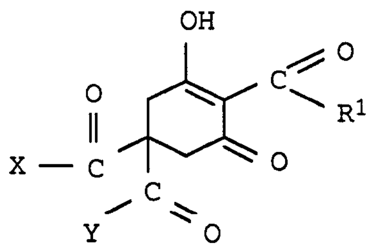

Ia

mit einem Amin der allgemeinen Formel V

$H_2N$-$R^3$ V

umsetzt.

**5.** Herbizides Mittel, enthaltend einen flüssigen oder festen Trägerstoff und mindestens ein Cyclohexenonderivat Ib gemäß Anspruch 3 oder ein landwirtschaftlich brauchbares Salz von Ib, oder einen Ester von Ib mit $C_1$-$C_{10}$-Carbonsäuren oder mit anorganischen Säuren.

**6.** Verfahren zur Bekämpfung von unerwünschtem Pflanzenwuchs, dadurch gekennzeichnet, daß man eine herbizid wirksamen Menge eines Cyclohexenonderivats der Formel Ib gemäß Anspruch 3, oder ein landwirtschaftlich brauchbares Salz von Ib oder einen Ester von Ib mit $C_1$-$C_{10}$-Carbonsäuren oder mit anorganischen Säuren, auf Pflanzen, deren Lebensraum oder auf Saatgut einwirken läßt.

**7.** Mittel zur Regulierung des Pflanzenwachstums, enthaltend einen flüssigen und/oder festen Trägerstoff und mindestens ein Cyclohexenonderivat der Formel Ia gemäß Anspruch 2, oder ein landwirtschaftlich brauchbares Salz von Ia oder einen Ester von Ia mit $C_1$-$C_{10}$-Carbonsäuren oder mit anorganischen Säuren.

**8.** Mittel zur Regulierung des Pflanzenwachstums, enthaltend einen flüssigen und/oder festen Trägerstoff und mindestens ein Cyclohexenonderivat der Formel Ic gemäß Anspruch 4, oder ein landwirtschaftlich brauchbares Salz von Ic oder einen Ester von Ic mit $C_1$-$C_{10}$-Carbonsäuren oder mit anorganischen Säuren.

**9.** Verfahren zur Regulierung des Pflanzenwachstums dadurch gekennzeichnet, daß man eine zum Regulieren des Pflanzenwachstums wirksame Menge eines Cyclohexenonderivats der Formel Ia gemäß Anspruch 2, oder ein landwirtschaftlich brauchbares Salz von Ia oder einen Ester von Ia mit $C_1$-$C_{10}$-Carbonsäuren oder mit anorganischen Säuren, auf Pflanzen, deren Lebensraum oder auf das Saatgut der Pflanzen einwirken läßt.

**10.** Verfahren zur Regulierung des Pflanzenwachstums dadurch gekennzeichnet, daß man eine zum Regulieren des Pflanzenwachstums wirksame Menge eines Cyclohexenonderivats der Formel Ic gemäß Anspruch 4, oder ein landwirtschaftlich brauchbares Salz von Ic oder einen Ester von Ic mit $C_1$-$C_{10}$-Carbonsäuren oder mit anorganischen Säuren, auf Pflanzen, deren Lebensraum oder auf das Saatgut der Pflanzen einwirken läßt.

**Patentansprüche für folgenden Vertragsstaat : ES**

1. Herbizides Mittel oder ein Mittel zur Regulierung des Pflanzenwachstums, enthaltend einen flüssigen und/oder festen Trägerstoff und mindestens ein Cyclohexenonderivat der allgemeinen Formel I

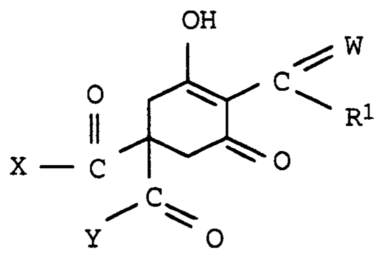

I

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy, $C_1$-$C_4$-Alkylsulfinyl und $C_1$-$C_4$-Alkylsulfonyl;

W Sauerstoff, eine Gruppe $=N-OR^2$ oder $=N-R^3$, mit

$R^2$

- einen $C_1$-$C_6$-Alkyl-, $C_3$-$C_6$-Alkenyl- oder $C_3$-$C_6$-Alkinylrest, wobei diese Reste noch einen bis drei der folgen den Substituenten tragen können, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;
- eine 3- bis 6-gliedrige Alkylkette oder eine 4- bis 6-gliedrige Alkenyl- oder Alkinylkette, wobei jeweils eine Kettenglied durch Sauerstoff, Schwefel oder eine Gruppe -SO-, -$SO_2$- oder -$N(R^8)$- ersetzt ist, und wobei die Kette noch zusätzlich ein bis drei Substituenten tragen kann, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;

$R^8$ steht für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl;

$R^3$

Wasserstoff, einen $C_1$-$C_6$-Alkylrest, einen Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, den Phenyl- oder Benzylrest, wobei die aromatischen Ringe noch ein bis drei Substituenten tragen können, ausgewählt aus einer Gruppe bestehend aus Nitro, Halogen, $C_1$-$C_4$-Alkyl,$C_1$-$C_4$-Halogenalkyl und $C_1$-$C_4$-Alkoxy;

X,Y eine Gruppe $-OR^4$ oder $-NR^5R^6$,

$R^4$

Wasserstoff, einen $C_1$-$C_6$-Alkyl-, $C_3$-$C_6$-Alkenyl-, $C_3$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_2$-alkyl-, $C_1$-$C_4$-Alkylthio-$C_1$-$C_2$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe -N($R^7$)- als Ringglied enthalten kann, wobei

$R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht,

sowie die landwirtschaftlich brauchbaren Salze von I und die Ester von I mit $C_1$-$C_{10}$-Carbonsäuren oder anorganischen Säuren.

**2.** Verfahren zur Herstellung von Cyclohexenonderivaten der Formel Ia

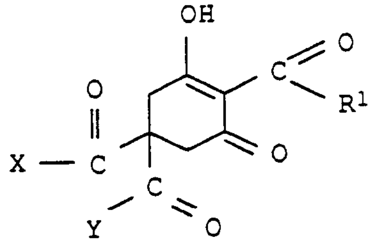

Ia

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl $C_1$-$C_4$-Alkoxy, $C_1$-$C_4$-Alkylsulfinyl und $C_1$-$C_4$-Alkylsulfonyl;

X,Y eine Gruppe -$OR^4$ oder -$NR^5R^6$, wobei

$R^4$

Wasserstoff, einen $C_1$-$C_6$-Alkyl-, $C_2$-$C_6$-Alkenyl-, $C_2$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$

Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, partiell oder vollständig halogeniertem $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe -N($R^7$)- als Ringglied enthalten kann, wobei

$R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht, bedeuten,

dadurch gekennzeichnet, daß man ein 5-substituiertes Cyclohexan-1,3-dion der allgemeinen Formel II

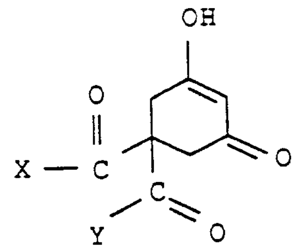

II

mit einem Säurederivat der allgemeinen Formel III,

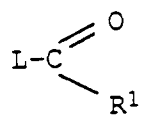

III

in der L für eine nukleophile Abgangsgruppe steht, umsetzt.

3. Verfahren zur Herstellung von Cyclohexenonderivaten der Formel

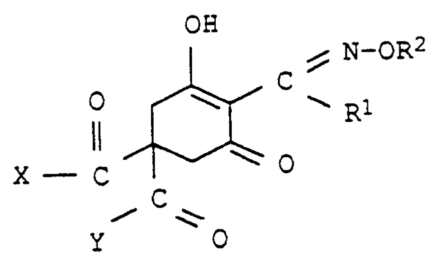

Ib

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl $C_1$-$C_4$-Alkoxy, $C_1$-$C_4$-Alkylsulfinyl und $C_1$-$C_4$-Alkylsulfonyl;

$R^2$ einen $C_1$-$C_6$-Alkyl-, $C_3$-$C_6$-Alkenyl- oder $C_3$-$C_6$-Alkinylrest, wobei diese Reste noch einen bis drei der folgenden Substituenten tragen können, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl und $C_1$-$C_4$-Alkoxy; eine 3- bis 6-gliedrige Alkylkette oder eine 4- bis 6-gliedrige Alkenyl- oder Alkinylkette, wobei jeweils eine Kettenglied durch Sauerstoff, Schwefel oder eine Gruppe -SO-, $-SO_2$- oder -N($R^8$)- ersetzt ist, und wobei die Kette noch zusätzlich ein bis drei Substituenten tragen kann, ausgewählt aus einer Gruppe bestehend aus Halogen, $C_1$-$C_4$-Alkyl, $C_1$-$C_4$-Alkoxy, Phenyl und 5- oder 6-gliedrigem Heteroaryl mit ein oder zwei Heteroatomen, ausgewählt aus einer Gruppe bestehend aus Stickstoff, Sauerstoff und Schwefel, wobei die Phenyl- und Heteroarylsubstituenten ihrerseits noch ein- bis drei Reste tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;

$R^8$ steht für Wasserstoff, $C_1$-$C_6$-Alkyl, $C_1$-$C_6$-Alkenyl, $C_1$-$C_6$-Alkinyl, $C_1$-$C_6$-Alkylcarbonyl oder Benzoyl;

X,Y eine Gruppe $-OR^4$ oder $-NR^5R^6$, wobei

$R^4$ Wasserstoff, einen $C_1$-$C_6$-Alkyl-, $C_2$-$C_6$-Alkenyl-, $C_2$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$ Wasserstoff, einen $C_1$-$C_6$-Alkyl-, $C_2$-$C_6$-Alkenyl-, $C_2$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$ Wasserstoff, einen $C_1$-$C_6$-Alkyl-, $C_2$-$C_6$-Alkenyl-, $C_2$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe $-N(R^7)$- als Ringglied enthalten kann, wobei

$R^7$ für Wasserstoff, $C_1$-$C_6$-Alkyl, $C_1$-$C_6$-Alkenyl, $C_1$-$C_6$-Alkinyl, $C_1$-$C_6$-Alkylcarbonyl oder Benzoyl steht,

bedeuten,

dadurch gekennzeichnet, daß man eine Verbindung der Formel Ia

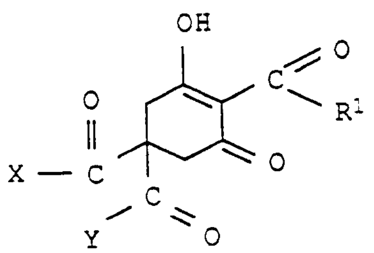

Ia

mit einem Hydroxylamin der allgemeinen Formel IV

$H_2N$-O-$R^2$ IV

umsetzt.

4. Verfahren zur Herstellung von Cyclohexenonderivaten der Formel

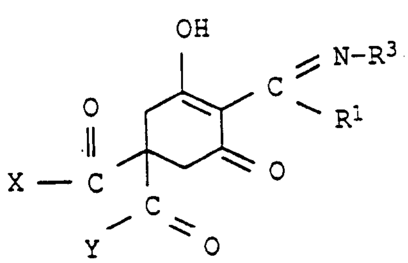

Ic

wobei die Variablen die folgende Bedeutung haben:

$R^1$ eine $C_1$-$C_{20}$-Alkylgruppe, eine $C_2$-$C_{20}$-Alkenylgruppe, eine $C_2$-$C_{20}$-Alkinylgruppe, eine $C_3$-$C_6$-Cycloalkylgruppe, eine $C_1$-$C_4$-Alkoxy-$C_1$-$C_4$-alkylgruppe, eine $C_1$-$C_4$-Alkylthio-$C_1$-$C_4$-alkylgruppe, die Phenyl- oder Benzylgruppe oder eine 5- oder 6-gliedrige Heteroaryl-Gruppe mit ein oder zwei Heteroatomen, ausgewählt aus einer Gruppe bestehend aus Stickstoff, Sauerstoff und Schwefel, wobei die aromatischen und heteroaromatischen Ringe noch ein- bis drei Reste tragen können, ausgewählt aus einer Gruppe bestehend aus Nitro, Cyano, Halogen, $C_1$-$C_4$-Alkyl, $C_1$-$C_4$-Halogenalkyl $C_1$-$C_4$-Alkoxy, $C_1$-$C_4$-Alkylsulfinyl und $C_1$-$C_4$-Alkylsulfonyl;

$R^3$ Wasserstoff, einen $C_1$-$C_6$-Alkylrest, einen Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, den Phenyl- oder Benzylrest, wobei die aromatischen Ringe noch ein bis drei Substituenten tragen können, ausgewählt aus einer Gruppe bestehend aus Nitro, Halogen, $C_1$-$C_4$-Alkyl, $C_1$-$C_4$-Halogenalkyl, $C_1$-$C_4$-Alkoxy und $C_1$-$C_4$-Halogenalkoxy;

X,Y eine Gruppe $-OR^4$ oder $-NR^5R^6$, wobei

$R^4$ Wasserstoff, einen $C_1$-$C_6$-Alkyl-, $C_3$-$C_6$-Alkenyl-, $C_3$-$C_6$-Alkinyl-, $C_1$-$C_4$-Alkoxy-$C_1$-$C_4$-alkyl- oder $C_1$-$C_4$-Alkylthio-$C_1$-$C_4$-alkylrest,

$R^5$ Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl- oder $C_1$-$C_4$-Alkylthio-$C_1$-$C_4$-alkylrest, und

$R^6$ Wasserstoff, einen $C_1$-$C_6$-Alkyl-, $C_3$-$C_6$-Alkenyl-, $C_3$-$C_6$-Alkinyl-, Hydroxy-$C_1$-$C_4$-alkyl-, $C_1$-$C_4$-Alkoxy-$C_1$-$C_4$-alkyl-, $C_1$-$C_4$-Alkylthio-$C_1$-$C_4$-alkyl- oder $C_1$-$C_6$-Alkylcarbonylrest, oder den Benzoylrest, der noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder noch einen bis drei Substituenten tragen kann, ausgewählt aus einer Gruppe bestehend aus Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio; oder

$R^5$ und $R^6$ zusammen mit dem Stickstoffatom, an das sie gebunden sind, einen 5- oder 6-gliedrigen Heterocyclus, der ein Sauerstoff- oder Schwefelatom oder eine Gruppe $-N(R^7)-$ als Ringglied enthalten kann, wobei

$R^7$ für Wasserstoff, $C_1$-$C_6$-Alkyl, $C_3$-$C_6$-Alkenyl, $C_3$-$C_6$-Alkinyl, $C_1$-$C_6$-Alkylcarbonyl oder Benzoyl steht,

bedeuten,

dadurch gekennzeichnet, daß man eine Verbindung der Formel Ia

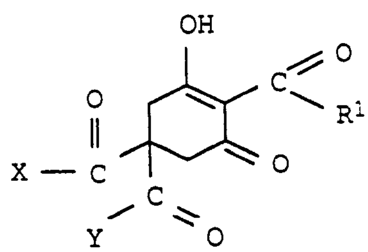

Ia

mit einem Amin der allgemeinen Formel V

$H_2N$-$R^3$ V

umsetzt.

5. Herbizides Mittel, enthaltend einen flüssigen oder festen Trägerstoff und mindestens ein Cyclohexenonderivat Ib gemäß Anspruch 3 oder ein landwirtschaftlich brauchbares Salz von Ib, oder einen Ester von Ib mit $C_1$-$C_{10}$-Carbonsäuren oder mit anorganischen Säuren

6. Verfahren zur Bekämpfung von unerwünschtem Pflanzenwuchs, dadurch gekennzeichnet, daß man eine herbizid wirksamen Menge eines Cyclohexenonderivats der Formel Ib gemäß Anspruch 3, oder ein landwirtschaftlich brauchbares Salz von Ib oder einen Ester von Ib mit $C_1$-$C_{10}$-Carbonsäuren oder mit anorganischen Säuren, auf pflanzen, deren Lebensraum oder auf Saatgut einwirken läßt.

7. Mittel zur Regulierung des Pflanzenwachstums, enthaltend einen flüssigen und/oder festen Trägerstoff und mindestens ein Cyclohexenonderivat der Formel Ia gemäß Anspruch 2, oder ein landwirtschaftlich brauchbares Salz von Ia oder einen Ester von Ia mit $C_1$-$C_{10}$-Carbonsäuren oder mit anorganischen Säuren.

**8.** Mittel zur Regulierung des Pflanzenwachstums, enthaltend einen flüssigen und/oder festen Trägerstoff und mindestens ein Cyclohexenonderivat der Formel Ic gemäß Anspruch 4, oder ein landwirtschaftlich brauchbares Salz von Ic oder einen Ester von Ic mit $C_1$-$C_{10}$-Carbonsäuren oder mit anorganischen Säuren.

**9.** Verfahren zur Regulierung des Pflanzenwachstums dadurch gekennzeichnet, daß man eine zum Regulieren des Pflanzenwachstums wirksame Menge eines Cyclohexenonderivats der Formel Ia gemäß Anspruch 2, oder ein landwirtschaftlich brauchbares Salz von Ia oder einen Ester von Ia mit $C_1$-$C_{10}$-Carbonsäuren oder mit anorganischen Säuren, auf Pflanzen, deren Lebensraum oder auf das Saatgut der Pflanzen einwirken läßt.

**10.** Verfahren zur Regulierung des Pflanzenwachstums dadurch gekennzeichnet, daß man eine zum Regulieren des Pflanzenwachstums wirksame Menge eines Cyclohexenonderivats der Formel Ic gemäß Anspruch 4, oder ein landwirtschaftlich brauchbares Salz von Ic oder einen Ester von Ic mit $C_1$-$C_{10}$-Carbonsäuren oder mit anorganischen Säuren, auf Pflanzen, deren Lebensraum oder auf das Saatgut der Pflanzen einwirken läßt.

## Claims

**Claims for the following Contracting States : AT, BE, CH, LI, DE, FR, GB, IT, NL**

**1.** A cyclohexenone derivative of the formula I

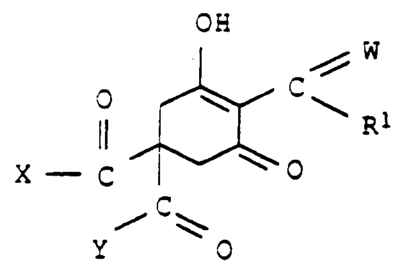

I

where

R¹ is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may, if desired, furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

W is oxygen, $=N\text{-}OR^2$ or $=N\text{-}R^3$, where

$R^2$ is $C_1$-$C_6$-alkyl, $C_3$-$C_6$-alkenyl or $C_3$-$C_6$-alkynyl, where these radicals may furthermore carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, and the phenyl and hetaryl substituents in turn may furthermore carry from one to three radicals selected from the group consisting of nitro, halogen, $C_1$-$C_4$-alkyl, $C_1$-$C_4$-haloalkyl, $C_1$-$C_4$-alkoxy and $C_1$-$C_4$-haloalkoxy;
a 3-membered to 6-membered alkyl chain or a 4-membered to 6-membered alkenyl or alkynyl chain, where one chain member in each case is replaced with oxygen, sulfur or -SO-, $-SO_2$- or $-N(R^8)$- and the chain may furthermore additionally carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, and the phenyl and hetaryl substituents may in turn furthermore carry from one to three radicals selected from the group consisting of nitro, halogen, $C_1$-$C_4$-alkyl, $C_1$-$C_4$-haloalkyl, $C_1$-$C_4$-alkoxy and $C_1$-$C_4$-haloalkoxy, where

$R^8$ is hydrogen, $C_1$-$C_6$-alkyl, $C_3$-$C_6$-alkenyl, $C_3$-$C_6$-alkynyl, $C_1$-$C_6$-alkylcarbonyl or benzoyl;

$R^3$ is hydrogen, $C_1$-$C_6$-alkyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl, phenyl or benzyl, where the aromatic rings may furthermore carry from one to three substituents selected from the group consisting of nitro, halogen, $C_1$-$C_4$-alkyl, $C_1$-$C_4$-haloalkyl and $C_1$-$C_4$-alkoxy;

X and Y are each $-OR^4$ or $-NR^5R^6$, where

$R^4$ is hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl or $C_1$-$C_4$-alkylthio-$C_1$-$C_4$-alkyl,

$R^5$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

$R^6$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$- or $C_2$-alkyl, $C_1$-$C_4$-alkylthio-$C_1$- or $C_2$-alkyl or $C_1$-$C_6$-alkylcarbonyl or benzoyl which may furthermore carry from one to three substituents selected from the group consisting of nitro, cyano, halogen, $C_1$-$C_4$-alkyl, $C_1$-$C_4$-haloalkyl, $C_1$-$C_4$-alkoxy and $C_1$-$C_4$-alkylthio, or

$R^5$ and $R^6$, together with the nitrogen atom to which they are bonded, may form a 5-membered or 6-membered heterocyclic structure which may contain an oxygen or sulfur atom or $-N(R^7)-$ as a ring member, where

$R^7$ is hydrogen, $C_1$-$C_6$-alkyl, $C_3$-$C_6$-alkenyl, $C_3$-$C_6$-alkynyl, $C_1$-$C_6$-alkylcarbonyl or benzoyl,

and the agriculturally useful salts of I and the esters of I with $C_1$-$C_{10}$-carboxylic acids or inorganic acids.

**2.** A process for the preparation of a cyclohexenone derivative of the formula Ia

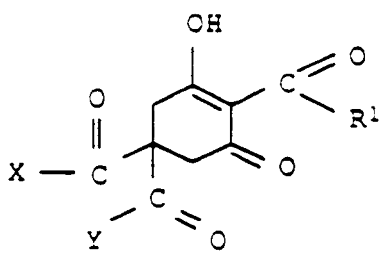

Ia

where

$R^1$ is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

X and Y are each $-OR^4$ or $-NR^5R^6$, where

$R^4$ is hydrogen, $C_1$-$C_6$-alkyl, $C_2$-$C_6$-alkenyl, $C_2$-$C_6$-alkynyl, $C_1$-$C_4$-alkoxy-$C_1$-$C_4$-alkyl or $C_1$-$C_4$-alkylthio-$C_1$-$C_4$-alkyl,

$R^5$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

$R^6$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl or $C_1$-$C_6$-alkylcarbonyl or benzoyl which may furthermore carry from one to three substituents selected from the group consisting of nitro, cyano, halogen, $C_1$-$C_4$-alkyl, partially or completely halogenated $C_1$-$C_4$-haloalkyl, $C_1$-$C_4$-alkoxy and $C_1$-$C_4$-alkylthio, or

$R^5$ and $R^6$, together with the nitrogen atom to which they are bonded, may form a 5-membered or 6-membered heterocyclic structure which may contain an oxygen or sulfur atom or $-N(R^7)-$ as a ring member, where

$R^7$ is hydrogen, $C_1$-$C_6$-alkyl, $C_3$-$C_6$-alkenyl, $C_3$-$C_6$-alkynyl, $C_1$-$C_6$-alkylcarbonyl or ben-

zoyl,

wherein a 5-substituted cyclohexane-1,3-dione of the formula II

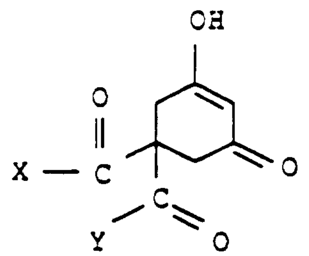

II

is reacted with an acid derivative of the formula III

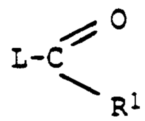

III

where L is a nucleophilic leaving group.

3. A process for the preparation of a cyclohexenone derivative of the formula

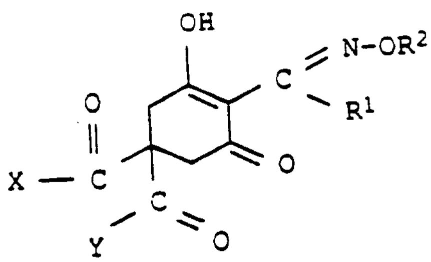

Ib

where

$R^1$ is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

$R^2$ is $C_1$-$C_6$-alkyl, $C_3$-$C_6$-alkenyl or $C_3$-$C_6$-alkynyl, where these radicals may furthermore carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, where the phenyl and hetaryl substituents in turn may furthermore carry from one to three radicals selected from the group consisting of nitro, halogen, $C_1$-$C_4$-alkyl, $C_1$-$C_4$-haloalkyl and $C_1$-$C_4$-alkoxy; a 3-membered to 6-membered alkyl chain or a 4-membered to 6-membered alkenyl or alkynyl chain, where one chain member in each case may be replaced with oxygen, sulfur or -SO-, $-SO_2-$ or $-N(R^8)-$, and the chain may furthermore additionally carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, where the phenyl and hetaryl substituents in turn may furthermore carry from one to three radicals selected from the group

consisting of nitro, halogen, $C_1$-$C_4$-alkyl, $C_1$-$C_4$-haloalkyl, $C_1$-$C_4$-alkoxy and $C_1$-$C_4$-haloalkoxy, where

$R^8$ is hydrogen, $C_1$-$C_6$-alkyl, $C_1$-$C_6$-alkenyl, $C_1$-$C_6$-alkynyl, $C_1$-$C_6$-alkylcarbonyl or benzoyl;

X and Y are each -$OR^4$ or -$NR^5R^6$, where

$R^4$ is hydrogen, $C_1$-$C_6$-alkyl, $C_2$-$C_6$-alkenyl, $C_2$-$C_6$-alkynyl, $C_1$-$C_4$-alkoxy-$C_1$-$C_4$-alkyl or $C_1$-$C_4$-alkylthio-$C_1$-$C_4$-alkyl,

$R^5$ is hydrogen, $C_1$-$C_6$-alkyl, $C_2$-$C_6$-alkenyl, $C_2$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

$R^6$ is hydrogen, $C_1$-$C_6$-alkyl, $C_2$-$C_6$-alkenyl, $C_2$-$C_6$-alkynyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl or $C_1$-$C_6$-alkylcarbonyl or benzoyl which may furthermore carry from one to three substituents selected from the group consisting of nitro, cyano, halogen, $C_1$-$C_4$-alkyl, $C_1$-$C_4$-haloalkyl, $C_1$-$C_4$-alkoxy and $C_1$-$C_4$-alkylthio, or

$R^5$ and $R^6$, together with the nitrogen atom to which they are bonded, are a 5-membered or 6-membered heterocyclic structure which may contain an oxygen or sulfur atom or -N-($R^7$)- as a ring member, where

$R^7$ is hydrogen, $C_1$-$C_6$-alkyl, $C_1$-$C_6$-alkenyl, $C_1$-$C_6$-alkynyl, $C_1$-$C_6$-alkylcarbonyl or benzoyl,

wherein a compound of the formula Ia

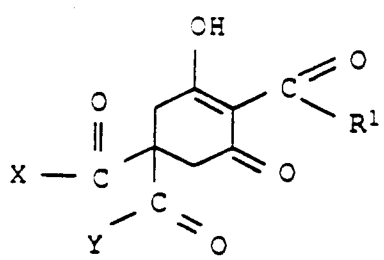

Ia

is reacted with a hydroxylamine of the formula IV

$H_2N$-O-$R^2$ IV

**4.** A process for the preparation of a cyclohexenone derivative of the formula

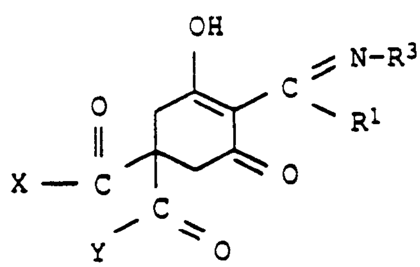

Ic

where

$R^1$ is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

$R^3$ is hydrogen, $C_1$-$C_6$-alkyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl, phenyl or benzyl, where the aromatic rings may furthermore carry from one to three substituents selected from the group consisting of nitro,

halogen, $C_1$-$C_4$-alkyl, $C_1$-$C_4$-haloalkyl, $C_1$-$C_4$-alkoxy and $C_1$-$C_4$-haloalkoxy;

X and Y are each $-OR^4$ or $-NR^5R^6$, where

$R^4$ is hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl or $C_1$-$C_4$-alkylthio-$C_1$-$C_4$-alkyl,

$R^5$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

$R^6$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl or $C_1$-$C_6$-alkylcarbonyl or benzoyl which may furthermore carry from one to three substituents selected from the group consisting of nitro, cyano, halogen, $C_1$-$C_4$-alkyl, $C_1$-$C_4$-haloalkyl, $C_1$-$C_4$-alkoxy and $C_1$-$C_4$-alkylthio, or

$R^5$ and $R^6$, together with the nitrogen atom to which they are bonded, are a 5-membered or 6-membered heterocyclic structure which may contain an oxygen or sulfur atom or $-N(R^7)-$ as a ring member, where

$R^7$ is hydrogen, $C_1$-$C_6$-alkyl, $C_3$-$C_6$-alkenyl, $C_3$-$C_6$-alkynyl, $C_1$-$C_6$-alkylcarbonyl or benzoyl,

wherein a compound of the formula Ia

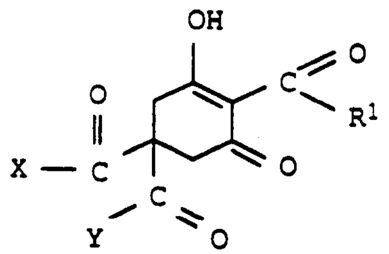

Ia

is reacted with an amine of the formula V

$H_2N$-$R^3$ V

5. A herbicide containing a liquid or solid carrier and at least one cyclohexenone derivative Ib as claimed in claim 3, or an agriculturally useful salt of Ib or an ester of Ib with $C_1$-$C_{10}$-carboxylic acids or with inorganic acids.

6. A method for controlling undesirable plant growth, wherein a herbicidal amount of a cyclohexenone derivative of the formula Ib as claimed in claim 3, or an agriculturally useful salt of Ib or an ester of Ib with $C_1$-$C_{10}$ carboxylic acids or with inorganic acids, is allowed to act on plants or their habitat or on seed.

7. A plant growth regulator containing a liquid or solid carrier and at least one cyclohexenone derivative of the formula Ia as claimed in claim 2, or an agriculturally useful salt of Ia or an ester of Ia with $C_1$-$C_{10}$-carboxylic acids or with inorganic acids.

8. A plant growth regulator containing a liquid or solid carrier and at least one cyclohexenone derivative of the formula Ic as claimed in claim 4, or an agriculturally useful salt of Ic or an ester of Ic with $C_1$-$C_{10}$-carboxylic acids or with inorganic acids.

9. A method for regulating plant growth, wherein an amount, effective for regulating plant growth, of a cyclohexenone derivative of the formula Ia as claimed in claim 2, or an agriculturally useful salt of Ia or an ester of Ia with $C_1$-$C_{10}$-carboxylic acids or with inorganic acids, is allowed to act on plants or their habitat or on the seed of the plants.

10. A method for regulating plant growth, wherein an amount, effective for regulating plant growth, of a cyclohexenone derivative of the formula Ic as claimed in claim 4, or an agriculturally useful salt of Ic or an ester of Ic with $C_1$-$C_{10}$-carboxylic acids or with inorganic acids, is allowed to act on plants or their habitat or on the seed of the plants.

**Claims for the following Contracting State : ES**

**1.** A herbicide or a plant growth regulator containing a liquid or solid carrier and at least one cyclohexenone derivative of the formula I

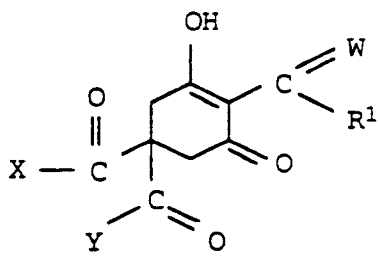

I

where

| | |
|---|---|
| $R^1$ | is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may, if desired, furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl; |
| W | is oxygen, $=N$-$OR^2$ or $=N$-$R^3$, where |
| $R^2$ | is $C_1$-$C_6$-alkyl, $C_3$-$C_6$-alkenyl or $C_3$-$C_6$-alkynyl, where these radicals may furthermore carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, and the phenyl and hetaryl substituents in turn may furthermore carry from one to three radicals selected from the group consisting of nitro, halogen, $C_1$-$C_4$-alkyl, $C_1$-$C_4$-haloalkyl, $C_1$-$C_4$-alkoxy and $C_1$-$C_4$-haloalkoxy;<br>a 3-membered to 6-membered alkyl chain or a 4-membered to 6-membered alkenyl or alkynyl chain, where one chain member in each case is replaced with oxygen, sulfur or -SO-, $-SO_2$- or -$N(R^8)$- and the chain may furthermore additionally carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, and the phenyl and hetaryl substituents may in turn furthermore carry from one to three radicals selected from the group consisting of nitro, halogen, $C_1$-$C_4$-alkyl, $C_1$-$C_4$-haloalkyl, $C_1$-$C_4$-alkoxy and $C_1$-$C_4$-haloalkoxy, where |
| $R^8$ | is hydrogen, $C_1$-$C_6$-alkyl, $C_3$-$C_6$-alkenyl, $C_3$-$C_6$-alkynyl, $C_1$-$C_6$-alkylcarbonyl or benzoyl; |
| $R^3$ | is hydrogen, $C_1$-$C_6$-alkyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl, phenyl or benzyl, where the aromatic rings may furthermore carry from one to three substituents selected from the group consisting of nitro, halogen, $C_1$-$C_4$-alkyl, $C_1$-$C_4$-haloalkyl and $C_1$-$C_4$-alkoxy; |
| X and Y | are each -$OR^4$ or -$NR^5R^6$, where |
| $R^4$ | is hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl or $C_1$-$C_4$-alkylthio-$C_1$-$C_4$-alkyl, |
| $R^5$ | is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and |
| $R^6$ | is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$- or $C_2$-alkyl, $C_1$-$C_4$-alkylthio-$C_1$- or $C_2$-alkyl or $C_1$-$C_6$-alkylcarbonyl or benzoyl which may furthermore carry from one to three substituents selected from the group consisting of nitro, cyano, halogen, $C_1$-$C_4$-alkyl, $C_1$-$C_4$-haloalkyl, $C_1$-$C_4$-alkoxy and $C_1$-$C_4$-alkylthio, or |
| $R^5$ and $R^6$, | together with the nitrogen atom to which they are bonded, may form a 5-membered or 6-membered heterocyclic structure which may contain an oxygen or sulfur atom or |

-N($R^7$)- as a ring member, where

$R^7$ is hydrogen, $C_1$-$C_6$-alkyl, $C_3$-$C_6$-alkenyl, $C_3$-$C_6$-alkynyl, $C_1$-$C_6$-alkylcarbonyl or benzoyl,

and the agriculturally useful salts of I and the esters of I with $C_1$-$C_{10}$-carboxylic acids or inorganic acids.

**2.** A process for the preparation of a cyclohexenone derivative of the formula Ia

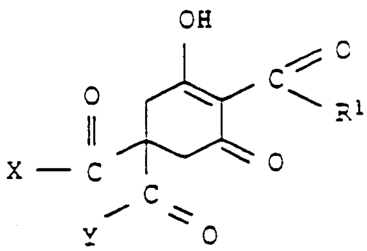

**Ia**

where

$R^1$ is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

X and Y are each -$OR^4$ or -$NR^5R^6$, where

$R^4$ is hydrogen, $C_1$-$C_6$-alkyl, $C_2$-$C_6$-alkenyl, $C_2$-$C_6$-alkynyl, $C_1$-$C_4$-alkoxy-$C_1$-$C_4$-alkyl or $C_1$-$C_4$-alkylthio-$C_1$-$C_4$-alkyl,

$R^5$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

$R^6$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl or $C_1$-$C_6$-alkylcarbonyl or benzoyl which may furthermore carry from one to three substituents selected from the group consisting of nitro, cyano, halogen, $C_1$-$C_4$-alkyl, partially or completely halogenated $C_1$-$C_4$-haloalkyl, $C_1$-$C_4$-alkoxy and $C_1$-$C_4$-alkylthio, or

$R^5$ and $R^6$, together with the nitrogen atom to which they are bonded, may form a 5-membered or 6-membered heterocyclic structure which may contain an oxygen or sulfur atom or -N($R^7$)- as a ring member, where

$R^7$ is hydrogen, $C_1$-$C_6$-alkyl, $C_3$-$C_6$-alkenyl, $C_3$-$C_6$-alkynyl, $C_1$-$C_6$-alkylcarbonyl or benzoyl,

wherein a 5-substituted cyclohexane-1,3-dione of the formula II

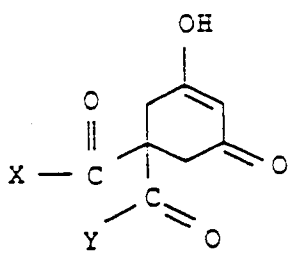

**II**

is reacted with an acid derivative of the formula III

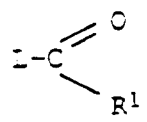

III

where L is a nucleophilic leaving group.

**3.** A process for the preparation of a cyclohexenone derivative of the formula

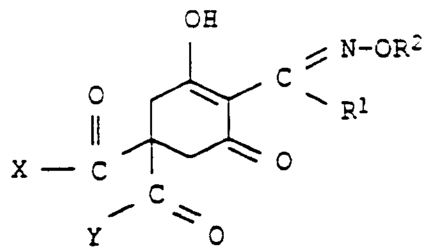

Ib

where

R[1] is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

R[2] is $C_1$-$C_6$-alkyl, $C_3$-$C_6$-alkenyl or $C_3$-$C_6$-alkynyl, where these radicals may furthermore carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, where the phenyl and hetaryl substituents in turn may furthermore carry from one to three radicals selected from the group consisting of nitro, halogen, $C_1$-$C_4$-alkyl, $C_1$-$C_4$-haloalkyl and $C_1$-$C_4$-alkoxy; a 3-membered to 6-membered alkyl chain or a 4-membered to 6-membered alkenyl or alkynyl chain, where one chain member in each case may be replaced with oxygen, sulfur or -SO-, $-SO_2-$ or $-N(R^8)-$, and the chain may furthermore additionally carry from one to three substituents selected from the group consisting of halogen, $C_1$-$C_4$-alkyl, $C_1$-$C_4$-alkoxy, phenyl and 5-membered or 6-membered hetaryl having one or two heteroatoms selected from the group consisting of nitrogen, oxygen and sulfur, where the phenyl and hetaryl substituents in turn may furthermore carry from one to three radicals selected from the group consisting of nitro, halogen, $C_1$-$C_4$-alkyl, $C_1$-$C_4$-haloalkyl, $C_1$-$C_4$-alkoxy and $C_1$-$C_4$-haloalkoxy, where

R[8] is hydrogen, $C_1$-$C_6$-alkyl, $C_1$-$C_6$-alkenyl, $C_1$-$C_6$-alkynyl, $C_1$-$C_6$-alkylcarbonyl or benzoyl;

X and Y are each $-OR^4$ or $-NR^5R^6$, where

R[4] is hydrogen, $C_1$-$C_6$-alkyl, $C_2$-$C_6$-alkenyl, $C_2$-$C_6$-alkynyl, $C_1$-$C_4$-alkoxy-$C_1$-$C_4$-alkyl or $C_1$-$C_4$-alkylthio-$C_1$-$C_4$-alkyl,

R[5] is hydrogen, $C_1$-$C_6$-alkyl, $C_2$-$C_6$-alkenyl, $C_2$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

R[6] is hydrogen, $C_1$-$C_6$-alkyl, $C_2$-$C_6$-alkenyl, $C_2$-$C_6$-alkynyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl or $C_1$-$C_6$-alkylcarbonyl or benzoyl which may furthermore carry from one to three substituents selected from the group consisting of nitro, cyano, halogen, $C_1$-$C_4$-alkyl, $C_1$-$C_4$-haloalkyl, $C_1$-$C_4$-alkoxy and $C_1$-$C_4$-alkylthio, or

$R^5$ and $R^6$, together with the nitrogen atom to which they are bonded, are a 5-membered or 6-membered heterocyclic structure which may contain an oxygen or sulfur atom or -N-($R^7$)- as a ring member, where

$R^7$ is hydrogen, $C_1$-$C_6$-alkyl, $C_1$-$C_6$-alkenyl, $C_1$-$C_6$-alkynyl, $C_1$-$C_6$-alkylcarbonyl or benzoyl,

wherein a compound of the formula Ia

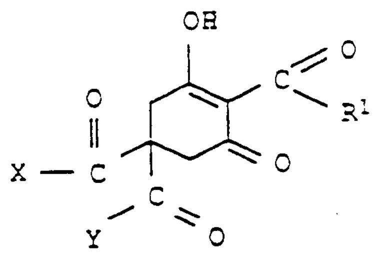

**Ia**

is reacted with a hydroxylamine of the formula IV

$H_2N$-O-$R^2$ IV

**4.** A process for the preparation of a cyclohexenone derivative of the formula

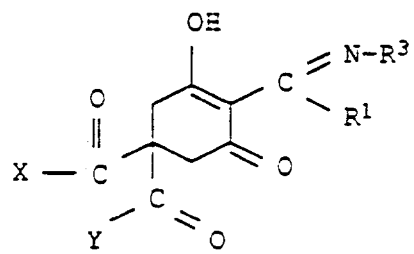

**Ic**

where

$R^1$ is $C_1$-$C_{20}$-alkyl, $C_2$-$C_{20}$-alkenyl, $C_2$-$C_{20}$-alkynyl, $C_3$-$C_6$-cycloalkyl, $C_1$-$C_4$-alkoxy-$C_1$-$C_4$-alkyl, $C_1$-$C_4$-alkylthio-$C_1$-$C_4$-alkyl, phenyl, benzyl or a 5-membered or 6-membered hetaryl group having one or two heteroatoms selected from the group consisting of nitrogen, oxygen and sulfur, where the aromatic and heteroaromatic rings may furthermore carry from one to three radicals selected from the group consisting of nitro, cyano, halogen, $C_1$-$C_4$-alkyl, $C_1$-$C_4$-haloalkyl, $C_1$-$C_4$-alkoxy, $C_1$-$C_4$-alkylsulfinyl and $C_1$-$C_4$-alkylsulfonyl;

$R^3$ is hydrogen, $C_1$-$C_6$-alkyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl, phenyl or benzyl, where the aromatic rings may furthermore carry from one to three substituents selected from the group consisting of nitro, halogen, $C_1$-$C_4$-alkyl, $C_1$-$C_4$-haloalkyl, $C_1$-$C_4$-alkoxy and $C_1$-$C_4$-haloalkoxy;

X and Y are each -$OR^4$ or -$NR^5R^6$, where

$R^4$ is hydrogen, $C_1$-$C_6$-alkyl, $C_3$-$C_6$-alkenyl, $C_3$-$C_6$-alkynyl, $C_1$-$C_4$-alkoxy-$C_1$-$C_4$-alkyl or $C_1$-$C_4$-alkylthio-$C_1$-$C_4$-alkyl,

$R^5$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl or $C_1$-$C_4$-alkylthio-$C_1$-$C_4$-alkyl and

$R^6$ is hydrogen, $C_1$-$C_6$-alkyl, $C_3$-$C_6$-alkenyl, $C_3$-$C_6$-alkynyl, hydroxy-$C_1$-$C_4$-alkyl, $C_1$-$C_4$-alkoxy-$C_1$-$C_4$-alkyl, $C_1$-$C_4$-alkylthio-$C_1$-$C_4$-alkyl or $C_1$-$C_6$-alkylcarbonyl or benzoyl which may furthermore carry from one to three substituents selected from the group consisting of nitro, cyano, halogen, $C_1$-$C_4$-alkyl, $C_1$-$C_4$-haloalkyl, $C_1$-$C_4$-alkoxy and $C_1$-$C_4$-alkylthio, or

$R^5$ and $R^6$, together with the nitrogen atom to which they are bonded, are a 5-membered or 6-membered heterocyclic structure which may contain an oxygen or sulfur atom or -N-($R^7$)- as a ring member, where

$R^7$ is hydrogen, $C_1$-$C_6$-alkyl, $C_3$-$C_6$-alkenyl, $C_3$-$C_6$-alkynyl, $C_1$-$C_6$-alkylcarbonyl or ben-

zoyl,
wherein a compound of the formula Ia

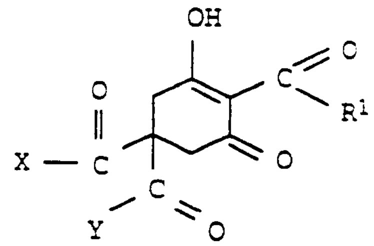

Ia

is reacted with an amine of the formula V

$H_2N\text{-}R^3$ V

5. A herbicide containing a liquid or solid carrier and at least one cyclohexenone derivative Ib as claimed in claim 3, or an agriculturally useful salt of Ib or an ester of Ib with $C_1$-$C_{10}$-carboxylic acids or with inorganic acids.

6. A method for controlling undesirable plant growth, wherein a herbicidal amount of a cyclohexenone derivative of the formula Ib as claimed in claim 3, or an agriculturally useful salt of Ib or an ester of Ib with $C_1$-$C_{10}$ carboxylic acids or with inorganic acids, is allowed to act on plants or their habitat or on seed.

7. A plant growth regulator containing a liquid or solid carrier and at least one cyclohexenone derivative of the formula Ia as claimed in claim 2, or an agriculturally useful salt of Ia or an ester of Ia with $C_1$-$C_{10}$-carboxylic acids or with inorganic acids.

8. A plant growth regulator containing a liquid or solid carrier and at least one cyclohexenone derivative of the formula Ic as claimed in claim 4, or an agriculturally useful salt of Ic or an ester of Ic with $C_1$-$C_{10}$-carboxylic acids or with inorganic acids.

9. A method for regulating plant growth, wherein an amount, effective for regulating plant growth, of a cyclohexenone derivative of the formula Ia as claimed in claim 2, or an agriculturally useful salt of Ia or an ester of Ia with $C_1$-$C_{10}$-carboxylic acids or with inorganic acids, is allowed to act on plants or their habitat or on the seed of the plants.

10. A method for regulating plant growth, wherein an amount, effective for regulating plant growth, of a cyclohexenone derivative of the formula Ic as claimed in claim 4, or an agriculturally useful salt of Ic or an ester of Ic with $C_1$-$C_{10}$-carboxylic acids or with inorganic acids, is allowed to act on plants or their habitat or on the seed of the plants.

**Revendications**

**Revendications pour les Etats contractants suivants : AT, BE, CH, LI, DE, FR, GB, IT, NL**

1. Dérivés de cyclohexénone de formule générale I

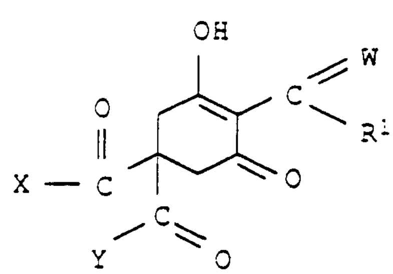

I

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogénoalkyle en $C_1$-$C_4$, alcoxy en $C_1$-$C_4$, alkylsulfinyle en $C_1$-$C_4$ et alkylsulfonyle en $C_1$-$C_4$;

W désigne l'oxygène, un groupe =N-$OR^2$ ou =N-$R^3$, avec

$R^2$ représentant

- un reste alkyle en $C_1$-$C_6$, alcényle en $C_3$-$C_6$ ou alcynyle en $C_3$-$C_6$, tandis que ces restes peuvent encore porter un à trois des substituants suivants, choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes, choisis dans un groupe consistant en l'azote, l'oxygène et le soufre, tandis que les substituants phényle et hétéroaryle peuvent à leur tour porter encore un à trois restes choisis dans un groupe consistant en un radical nitro, halogéno, alkyle en $C_1$-$C_4$,halogénoalkyle en $C_1$-$C_4$, alcoxy en $C_1$-$C_4$ et halogénoalcoxy en $C_1$-$C_4$;
- une chaîne alkyle à 3 à 6 chaînons ou une chaîne alcényle ou alcynyle à 4 à 6 chaînons, tandis que dans chaque cas un terme de la chaîne peut être remplacé par de l'oxygène, du soufre ou un groupe -SO-, -$SO_2$- ou -N($R^8$)-, et tandis que la chaîne peut encore porter en outre un à trois substituants choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes choisis dans un groupe consistant en l'azote, l'oxygène et le soufre, tandis que les substituants phényle et hétéroaryle peuvent quant à eux porter encore un à trois restes choisis dans un groupe consistant en un radical nitro, halogéno, alkyle en $C_1$-$C_4$, halogénoalkyle en $C_1$-$C_4$, alcoxy en $C_1$-$C_4$ et halogénoalcoxy en $C_1$-$C_4$;

$R^8$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle;

$R^3$ représentant
l'hydrogène, un reste alkyle en $C_1$-$C_6$, un reste hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, le reste phényle ou benzyle, tandis que les cycles aromatiques peuvent encore porter un à trois substituants choisis dans un groupe consistant en un radical nitro, halogéno, alkyle en $C_1$-$C_4$, halogénoalkyle en $C_1$-$C_4$ et alcoxy en $C_1$-$C_4$;

X, Y désignant un groupe -$OR^4$ ou -$NR^5R^6$, avec

$R^4$
hydrogène ou un reste alkyle en $C_1$-$C_6$, alcényle en $C_3$-$C_6$, alcynyle en $C_3$-$C_6$, alcoxy($C_1$-$C_4$)alkyle en $C_1$-$C_4$ ou alkylthio($C_1$-$C_4$)alkyle en $C_1$-$C_4$,

$R^5$
hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$
hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_2$ ou alkylthio($C_1$-$C_4$)alkyle en $C_1$-$C_2$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$, alcoxy en $C_1$-$C_4$ et alkylthio en $C_1$-$C_4$; ou
$R^5$ et $R^6$, conjointement avec l'atome d'azote auquel ils sont liés, un hétérocycle à 5 ou 6 chaînons, qui peut contenir un atome d'oxygène ou de soufre ou un groupe -N($R^7$)- comme chaînon du cycle, tandis que

$R^7$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle,

ainsi que les sels de I utilisables en agriculture et les esters de I avec des acides carboxyliques en $C_1$-$C_{10}$ ou des acides inorganiques.

**2.** Procédé pour la préparation de dérivés de cyclohexénone de formule Ia

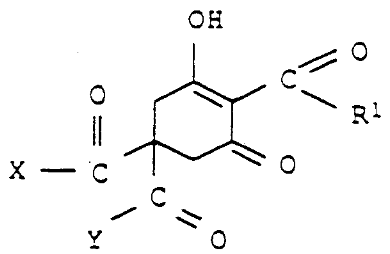

Ia

où les variables ont la signification suivante:

- $R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)-alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogéno-alkyle en $C_1$-$C_4$, alcoxy en $C_1$-$C_4$, alkylsulfinyle en $C_1$-$C_4$ et alkylsulfonyle en $C_1$-$C_4$;
- X, Y désignent un groupe $-OR^4$ ou $-NR^5R^6$, avec
- $R^4$ hydrogène ou un reste alkyle en $C_1$-$C_6$, alcényle en $C_2$-$C_6$, alcynyle en $C_2$-$C_6$, alcoxy($C_1$-$C_4$)alkyle en $C_1$-$C_4$ ou alkylthio($C_1$-$C_4$)alkyle en $C_1$-$C_4$,
- $R^5$ hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et
- $R^6$ hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$ partiellement ou totalement halogéné, alcoxy en $C_1$-$C_4$ et alkylthio en $C_1$-$C_4$; ou
  $R^5$ et $R^6$, conjointement avec l'atome d'azote auquel ils sont liés, un hétérocycle à 5 ou 6 chaînons, qui peut contenir un atome d'oxygène ou de soufre ou un groupe $-N(R^7)-$ comme chaînon du cycle, tandis que
- $R^7$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle,

caractérisé en ce qu'on fait réagir une cyclohexane-1,3-dione 5-substituée de formule générale II

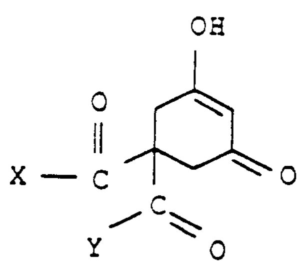

II

avec un dérivé d'acide de formule générale III,

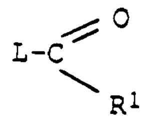

III

dans laquelle L représente un groupe séparable nucléophile.

**3.** Procédé pour la préparation de dérivés de cyclohexénone de formule

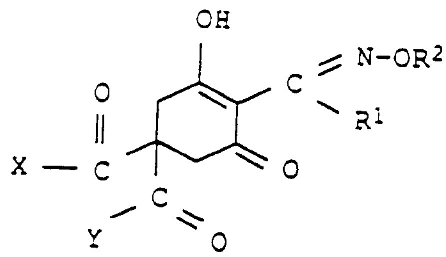

Ib

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogénoalkyle en $C_1$-$C_4$, alcoxy en $C_1$-$C_4$, alkylsulfinyle en $C_1$-$C_4$ et alkylsulfonyle en $C_1$-$C_4$;

$R^2$ représente un reste alkyle en $C_1$-$C_6$, alcényle en $C_3$-$C_6$ ou alcynyle en $C_3$-$C_6$, tandis que ces restes peuvent encore porter un à trois des substituants suivants, choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes, choisis dans un groupe consistant en l'azote, l'oxygène et le soufre, tandis que les substituants phényle et hétéroaryle peuvent à leur tour porter encore un à trois restes choisis dans un groupe consistant en un radical nitro, halogéno, alkyle en $C_1$-$C_4$,halogénoalkyle en $C_1$-$C_4$ et alcoxy en $C_1$-$C_4$; une chaîne alkyle à 3 à 6 chaînons ou une chaîne alcényle ou alcynyle à 4 à 6 chaînons, tandis que dans chaque cas un terme de la chaîne peut être remplacé par de l'oxygène, du soufre ou un groupe -SO-, $-SO_2-$ ou $-N(R^8)-$, et tandis que la chaîne peut encore porter en outre un à trois substituants choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes choisis dans un groupe consistant en l'azote, l'oxygène et le soufre, tandis que les substituants phényle et hétéroaryle peuvent quant à eux porter encore un à trois restes choisis dans un groupe consistant en un radical nitro, halogéno, alkyle en $C_1$-$C_4$, halogénoalkyle en $C_1$-$C_4$, alcoxy en $C_1$-$C_4$ et halogénoalcoxy en $C_1$-$C_4$;

$R^8$ désigne l'hydrogène ou un groupe alkyle en $C_1$-$C_6$, alcényle en $C_1$-$C_6$, alcynyle en $C_1$-$C_6$, alkyl($C_1$-$C_6$)carbonyle ou benzoyle;

X, Y désignent un groupe $-OR^4$ ou $-NR^5R^6$, avec

$R^4$

hydrogène ou un reste alkyle en $C_1$-$C_6$, alcényle en $C_2$-$C_6$, alcynyle en $C_2$-$C_6$, alcoxy($C_1$-$C_4$)alkyle en $C_1$-$C_4$ ou alkylthio($C_1$-$C_4$)alkyle en $C_1$-$C_4$,

$R^5$

hydrogène ou un reste alkyle en $C_1$-$C_6$, alcényle en $C_2$-$C_6$, alcynyle en $C_2$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$

hydrogène ou un reste alkyle en $C_1$-$C_6$, alcényle en $C_2$-$C_6$, alcynyle en $C_2$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$ ou alkyl($C_1$-

$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$, alcoxy en $C_1$-$C_4$ et alkylthio en $C_1$-$C_4$; ou

$R^5$ et $R^6$, conjointement avec l'atome d'azote auquel ils sont liés, un hétérocycle à 5 ou 6 chaînons, qui peut contenir un atome d'oxygène ou de soufre ou un groupe -N($R^7$)- comme chaînon du cycle, tandis que

$R^7$ désigne l'hydrogène ou un groupe alkyle en $C_1$-$C_6$, alcényle en $C_1$-$C_6$, alcynyle en $C_1$-$C_6$, alkyl($C_1$-$C_6$)carbonyle ou benzoyle,

caractérisé en ce qu'on fait réagir un composé de formule Ia

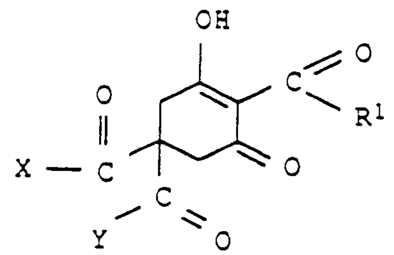

Ia

avec une hydroxylamine de formule générale IV

$H_2N$-O-$R^2$ IV.

**4.** Procédé pour la préparation de dérivés de cyclohexénone de formule

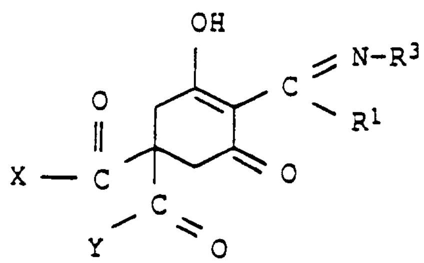

Ic

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogénoalkyle en $C_1$-$C_4$, alcoxy en $C_1$-$C_4$, alkylsulfinyle en $C_1$-$C_4$ et alkylsulfonyle en $C_1$-$C_4$;

$R^3$ représente
l'hydrogène, un reste alkyle en $C_1$-$C_6$, un reste hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, le reste phényle ou benzyle, tandis que les cycles aromatiques peuvent encore porter un à trois substituants choisis dans un groupe consistant en un radical nitro, halogéno, alkyle en $C_1$-$C_4$, halogénoalkyle en $C_1$-$C_4$, alcoxy en $C_1$-$C_4$ et halogénoalcoxy en $C_1$-$C_4$;

X, Y désignent un groupe -$OR^4$ ou -$NR^5R^6$, avec

$R^4$
hydrogène ou un reste alkyle en $C_1$-$C_6$, alcényle en $C_3$-$C_6$, alcynyle en $C_3$-$C_6$, alcoxy($C_1$-$C_4$)alkyle en $C_1$-$C_4$ ou alkylthio($C_1$-$C_4$)alkyle en $C_1$-$C_4$,

$R^5$
hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$ hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$, alcoxy en $C_1$-$C_4$ et alkylthio en $C_1$-$C_4$; ou

$R^5$ et $R^6$, conjointement avec l'atome d'azote auquel ils sont liés, un hétérocycle à 5 ou 6 chaînons, qui peut contenir un atome d'oxygène ou de soufre ou un groupe -N($R^7$)- comme chaînon du cycle, tandis que

$R^7$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle,

caractérisé en ce qu'on fait réagir un composé de formule Ia

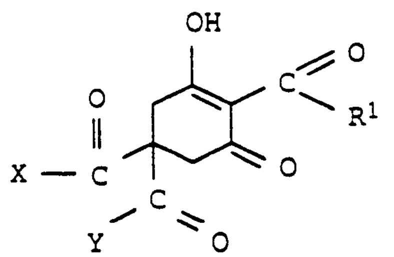

Ia

avec une amine de formule générale V

$H_2N$-$R^3$ V.

**5.** Agent herbicide, contenant un vecteur liquide ou solide et au moins un dérivé de cyclohexénone Ib selon la revendication 3 ou un sel de Ib utilisable en agriculture, ou un ester de Ib avec des acides carboxyliques en $C_1$-$C_{10}$ ou avec des acides inorganiques.

**6.** Procédé pour la lutte contre la croissance des plantes parasites, caractérisé en ce qu'on fait agir une quantité à activité herbicide d'un dérivé de cyclohexénone de formule Ib selon la revendication 3, ou un sel de Ib utilisable en agriculture ou un ester de Ib avec des acides carboxyliques en $C_1$-$C_{10}$ ou avec des acides inorganiques, sur des plantes, leur espace vital ou les semences.

**7.** Agent pour la régulation de la croissance des plantes, contenant un vecteur liquide et/ou solide et au moins un dérivé de cyclohexénone de formule Ia selon la revendication 2, ou un sel de Ia utilisable en agriculture ou un ester de Ia avec des acides carboxyliques en $C_1$-$C_{10}$ ou avec des acides inorganiques.

**8.** Agent pour la régulation de la croissance des plantes, contenant un vecteur liquide et/ou solide et au moins un dérivé de cyclohexénone de formule Ic selon la revendication 4, ou un sel de Ic utilisable en agriculture ou un ester de Ic avec des acides carboxyliques en $C_1$-$C_{10}$ ou avec des acides inorganiques.

**9.** Procédé pour la régulation de la croissance des plantes, caractérisé en ce qu'on fait agir une quantité efficace pour la régulation de la croissance des plantes d'un dérivé de cyclohexénone de formule Ia selon la revendication 2 ou un sel de Ia utilisable en agriculture ou un ester de Ia avec des acides carboxyliques en $C_1$-$C_{10}$ ou avec des acides inorganiques, sur des plantes, leur espace vital ou les semences.

**10.** Procédé pour la régulation de la croissance des plantes, caractérisé en ce qu'on fait agir une quantité efficace pour la régulation de la croissance des plantes d'un dérivé de cyclohexénone de formule Ic selon la revendication 4 ou un sel de Ic utilisable en agriculture ou un ester de Ic avec des acides carboxyliques en $C_1$-$C_{10}$ ou avec des acides inorganiques, sur des plantes, leur espace vital ou les semences.

**Revendications pour l'Etat contractant suivant : ES**

1. Agent herbicide ou agent pour la régulation de la croissance des plantes, contenant un vecteur liquide et/ou solide et au moins un dérivé de cyclohexénone de formule générale I

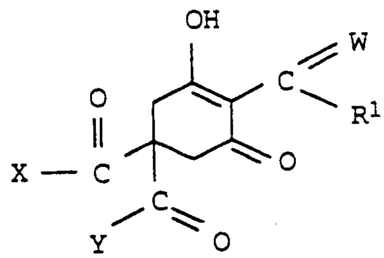

I

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogénoalkyle en $C_1$-$C_4$, alcoxy en $C_1$-$C_4$, alkylsulfinyle en $C_1$-$C_4$ et alkylsulfonyle en $C_1$-$C_4$;

W désigne l'oxygène, un groupe $=N-OR^2$ ou $=N-R^3$, avec

$R^2$ représentant

- un reste alkyle en $C_1$-$C_6$, alcényle en $C_3$-$C_6$ ou alcynyle en $C_3$-$C_6$, tandis que ces restes peuvent encore porter un à trois des substituants suivants, choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes, choisis dans un groupe consistant en l'azote, l'oxygène et le soufre, tandis que les substituants phényle et hétéroaryle peuvent à leur tour porter encore un à trois restes choisis dans un groupe consistant en un radical nitro, halogéno, alkyle en $C_1$-$C_4$,halogénoalkyle en $C_1$-$C_4$, alcoxy en $C_1$-$C_4$ et halogénoalcoxy en $C_1$-$C_4$;
- une chaîne alkyle à 3 à 6 chaînons ou une chaîne alcényle ou alcynyle à 4 à 6 chaînons, tandis que dans chaque cas un terme de la chaîne peut être remplacé par de l'oxygène, du soufre ou un groupe -SO-, $-SO_2-$ ou $-N(R^8)-$, et tandis que la chaîne peut encore porter en outre un à trois substituants choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes choisis dans un groupe consistant en l'azote, l'oxygène et le soufre, tandis que les substituants phényle et hétéroaryle peuvent quant à eux porter encore un à trois restes choisis dans un groupe consistant en un radical nitro, halogéno, alkyle en $C_1$-$C_4$, halogénoalkyle en $C_1$-$C_4$, alcoxy en $C_1$-$C_4$ et halogénoalcoxy en $C_1$-$C_4$;

$R^8$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle;

$R^3$ représentant

l'hydrogène, un reste alkyle en $C_1$-$C_6$, un reste hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, le reste phényle ou benzyle, tandis que les cycles aromatiques peuvent encore porter un à trois substituants choisis dans un groupe consistant en un radical nitro, halogéno, alkyle en $C_1$-$C_4$, halogénoalkyle en $C_1$-$C_4$ et alcoxy en $C_1$-$C_4$;

X, Y désignant un groupe $-OR^4$ ou $-NR^5R^6$, avec

$R^4$

hydrogène ou un reste alkyle en $C_1$-$C_6$, alcényle en $C_3$-$C_6$, alcynyle en $C_3$-$C_6$, alcoxy($C_1$-$C_4$)alkyle en $C_1$-$C_4$ ou alkylthio($C_1$-$C_4$)alkyle en $C_1$-$C_4$,

$R^5$

hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$

hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_2$ ou alkylthio($C_1$-$C_4$)alkyle en $C_1$-$C_2$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$, alcoxy en $C_1$-$C_4$ et alkylthio en $C_1$-$C_4$; ou

$R^5$ et $R^6$, conjointement avec l'atome d'azote auquel ils sont liés, un hétérocycle à 5 ou 6 chaînons, qui peut contenir un atome d'oxygène ou de soufre ou un groupe -N($R^7$)- comme chaînon du cycle, tandis que

$R^7$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle,

ainsi que les sels de I utilisables en agriculture et les esters de I avec des acides carboxyliques en $C_1$-$C_{10}$ ou des acides inorganiques.

**2.** Procédé pour la préparation de dérivés de cyclohexénone de formule Ia

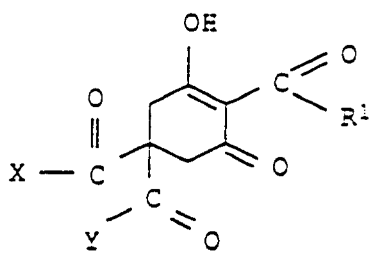

Ia

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogéno-alkyle en $C_1$-$C_4$, alcoxy en $C_1$-$C_4$, alkylsulfinyle en $C_1$-$C_4$ et alkylsulfonyle en $C_1$-$C_4$;

X, Y désignent un groupe -$OR^4$ ou -$NR^5R^6$, avec

$R^4$

hydrogène ou un reste alkyle en $C_1$-$C_6$, alcényle en $C_2$-$C_6$, alcynyle en $C_2$-$C_6$, alcoxy($C_1$-$C_4$)alkyle en $C_1$-$C_4$ ou alkylthio($C_1$-$C_4$)alkyle en $C_1$-$C_4$,

$R^5$

hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$

hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$ partiellement ou totalement halogéné, alcoxy en $C_1$-$C_4$ et alkylthio en $C_1$-$C_4$; ou

$R^5$ et $R^6$, conjointement avec l'atome d'azote auquel ils sont liés, un hétérocycle à 5 ou 6 chaînons, qui peut contenir un atome d'oxygène ou de soufre ou un groupe -N($R^7$)- comme chaînon du cycle, tandis que

$R^7$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle,

caractérisé en ce qu'on fait réagir une cyclohexane-1,3-dione 5-substituée de formule générale II

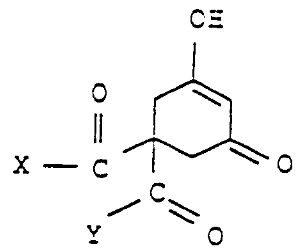

II

avec un dérivé d'acide de formule générale III,

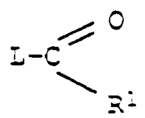

III

dans laquelle L représente un groupe séparable nucléophile.

3. Procédé pour la préparation de dérivés de cyclohexénone de formule

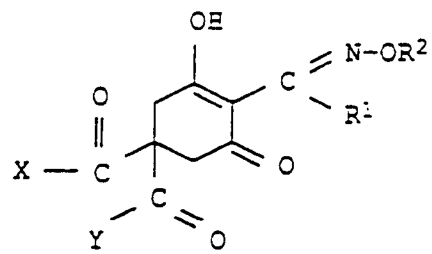

Ib

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogénoalkyle en $C_1$-$C_4$, alcoxy en $C_1$-$C_4$, alkylsulfinyle en $C_1$-$C_4$ et alkylsulfonyle en $C_1$-$C_4$;

$R^2$ représente un reste alkyle en $C_1$-$C_6$, alcényle en $C_3$-$C_6$ ou alcynyle en $C_3$-$C_6$, tandis que ces restes peuvent encore porter un à trois des substituants suivants, choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes, choisis dans un groupe consistant en l'azote, l'oxygène et le soufre, tandis que les substituants phényle et hétéroaryle peuvent à leur tour porter encore un à trois restes choisis dans un groupe consistant en un radical nitro, halogéno, alkyle en $C_1$-$C_4$,halogénoalkyle en $C_1$-$C_4$ et alcoxy en $C_1$-$C_4$; une chaîne alkyle à 3 à 6 chaînons ou une chaîne alcényle ou alcynyle à 4 à 6 chaînons, tandis que dans chaque cas un terme de la chaîne peut être remplacé par de l'oxygène, du soufre ou un groupe -SO-, $-SO_2-$ ou $-N(R^8)-$, et tandis que la chaîne peut encore porter en outre un à trois substituants choisis dans un groupe consistant en un radical halogéno, alkyle en $C_1$-$C_4$, alcoxy en $C_1$-$C_4$, phényle et hétéroaryle à 5 ou 6 chaînons ayant un ou deux hétéroatomes choisis dans un groupe consistant en l'azote, l'oxygène et le soufre, tandis que les substituants phényle et hétéroaryle peuvent quant à eux porter encore un à trois restes choisis dans un groupe consistant en un radical nitro, halogéno, alkyle en $C_1$-$C_4$, halogénoalkyle en $C_1$-$C_4$, alcoxy en $C_1$-$C_4$ et halogénoalcoxy en $C_1$-$C_4$;

$R^8$ désigne l'hydrogène ou un groupe alkyle en $C_1$-$C_6$, alcényle en $C_1$-$C_6$, alcynyle en $C_1$-$C_6$, alkyl($C_1$-$C_6$)carbonyle ou benzoyle;

X, Y désignent un groupe -$OR^4$ ou -$NR^5R^6$, avec

$R^4$ hydrogène ou un reste alkyle en $C_1$-$C_6$, alcényle en $C_2$-$C_6$, alcynyle en $C_2$-$C_6$, alcoxy($C_1$-$C_4$)alkyle en $C_1$-$C_4$ ou alkylthio($C_1$-$C_4$)alkyle en $C_1$-$C_4$,

$R^5$ hydrogène ou un reste alkyle en $C_1$-$C_6$, alcényle en $C_2$-$C_6$, alcynyle en $C_2$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$ hydrogène ou un reste alkyle en $C_1$-$C_6$, alcényle en $C_2$-$C_6$, alcynyle en $C_2$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$, alcoxy en $C_1$-$C_4$ et alkylthio en $C_1$-$C_4$; ou

$R^5$ et $R^6$, conjointement avec l'atome d'azote auquel ils sont liés, un hétérocycle à 5 ou 6 chaînons, qui peut contenir un atome d'oxygène ou de soufre ou un groupe -N($R^7$)- comme chaînon du cycle, tandis que

$R^7$ désigne l'hydrogène ou un groupe alkyle en $C_1$-$C_6$, alcényle en $C_1$-$C_6$, alcynyle en $C_1$-$C_6$, alkyl($C_1$-$C_6$)carbonyle ou benzoyle,

caractérisé en ce qu'on fait réagir un composé de formule Ia

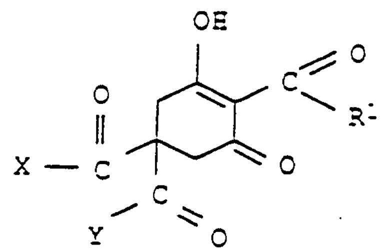

Ia

avec une hydroxylamine de formule générale IV

$H_2N$-O-$R^2$ IV.

**4.** Procédé pour la préparation de dérivés de cyclohexénone de formule

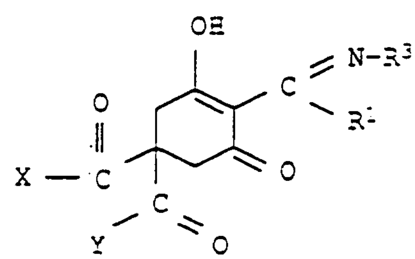

Ic

où les variables ont la signification suivante:

$R^1$ représente un groupe alkyle en $C_1$-$C_{20}$, un groupe alcényle en $C_2$-$C_{20}$, un groupe alcynyle en $C_2$-$C_{20}$, un groupe cycloalkyle en $C_3$-$C_6$, un groupe alcoxy($C_1$-$C_4$)alkyle en $C_1$-$C_4$, un groupe alkylthio($C_1$-$C_4$)alkyle en $C_1$-$C_4$, le groupe phényle ou benzyle ou un groupe hétéroaryle à 5 ou 6 chaînons ayant un ou deux hétéroatomes, choisis dans un groupe consistant en l'azote, l'oxygène et le soufre, tandis que les cycles aromatiques et hétéroaromatiques peuvent encore porter un à trois restes, choisis dans un groupe consistant en un radical nitro, cyano, halogéno, alkyle en $C_1$-$C_4$, halogénoalkyle en $C_1$-$C_4$, alcoxy en $C_1$-$C_4$, alkylsulfinyle en $C_1$-$C_4$ et alkylsulfonyle en $C_1$-$C_4$;

$R^3$ représente

l'hydrogène, un reste alkyle en $C_1$-$C_6$, un reste hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, le reste phényle ou benzyle, tandis que les cycles aromatiques peuvent encore porter un à trois substituants choisis dans un groupe consistant en un radical nitro, halogéno, alkyle en $C_1$-$C_4$, halogénoalkyle en $C_1$-$C_4$, alcoxy en $C_1$-$C_4$ et halogénoalcoxy en $C_1$-$C_4$;

X, Y désignent un groupe $-OR^4$ ou $-NR^5R^6$, avec

$R^4$ hydrogène ou un reste alkyle en $C_1$-$C_6$, alcényle en $C_3$-$C_6$, alcynyle en $C_3$-$C_6$, alcoxy($C_1$-$C_4$)alkyle en $C_1$-$C_4$ ou alkylthio($C_1$-$C_4$)alkyle en $C_1$-$C_4$,

$R^5$ hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$, et

$R^6$ hydrogène ou un reste alkyle en $C_1$-$C_6$, alcényle en $C_3$-$C_6$, alcynyle en $C_3$-$C_6$, hydroxyalkyle en $C_1$-$C_4$, alcoxy($C_1$-$C_4$)alkyle en $C_1$-$C_4$ ou alkylthio($C_1$-$C_4$)alkyle en $C_1$-$C_4$ ou alkyl($C_1$-$C_6$)carbonyle, ou le reste benzoyle, qui peut encore porter un à trois substituants choisis dans un groupe consistant en un radical nitro, cyano, halogéno, alkyle en $C_1$-$C_4$, halogénoalkyle en $C_1$-$C_4$, alcoxy en $C_1$-$C_4$ et alkylthio en $C_1$-$C_4$; ou

$R^5$ et $R^6$, conjointement avec l'atome d'azote auquel ils sont liés, un hétérocycle à 5 ou 6 chaînons, qui peut contenir un atome d'oxygène ou de soufre ou un groupe $-N(R^7)-$ comme chaînon du cycle, tandis que

$R^7$ désigne l'hydrogène ou un groupe alkyle en $C_1$-$C_6$, alcényle en $C_3$-$C_6$, alcynyle en $C_3$-$C_6$, alkyl($C_1$-$C_6$)carbonyle ou benzoyle,

caractérisé en ce qu'on fait réagir un composé de formule Ia

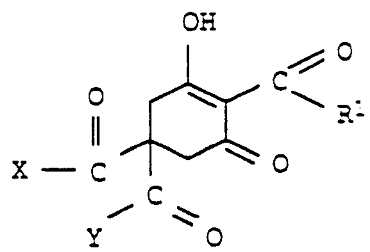

Ia

avec une amine de formule générale V

$H_2N-R^3$ V.

5. Agent herbicide, contenant un vecteur liquide ou solide et au moins un dérivé de cyclohexénone Ib selon la revendication 3 ou un sel de Ib utilisable en agriculture, ou un ester de Ib avec des acides carboxyliques en $C_1$-$C_{10}$ ou avec des acides inorganiques.

6. Procédé pour la lutte contre la croissance des plantes parasites, caractérisé en ce qu'on fait agir une quantité à activité herbicide d'un dérivé de cyclohexénone de formule Ib selon la revendication 3, ou un sel de Ib utilisable en agriculture ou un ester de Ib avec des acides carboxyliques en $C_1$-$C_{10}$ ou avec des acides inorganiques, sur des plantes, leur espace vital ou les semences.

7. Agent pour la régulation de la croissance des plantes, contenant un vecteur liquide et/ou solide et au moins un dérivé de cyclohexénone de formule Ia selon la revendication 2, ou un sel de Ia utilisable en agriculture ou un ester de Ia avec des acides carboxyliques en $C_1$-$C_{10}$ ou avec des acides inorganiques.

8. Agent pour la régulation de la croissance des plantes, contenant un vecteur liquide et/ou solide et au moins un dérivé de cyclohexénone de formule Ic selon la revendication 4, ou un sel de Ic utilisable en agriculture ou un ester de Ic avec des acides carboxyliques en $C_1$-$C_{10}$ ou avec des acides inorganiques.

**9.** Procédé pour la régulation de la croissance des plantes, caractérisé en ce qu'on fait agir une quantité efficace pour la régulation de la croissance des plantes d'un dérivé de cyclohexénone de formule Ia selon la revendication 2 ou un sel de Ia utilisable en agriculture ou un ester de Ia avec des acides carboxyliques en $C_1$-$C_{10}$ ou avec des acides inorganiques, sur des plantes, leur espace vital ou les semences.

**10.** Procédé pour la régulation de la croissance des plantes, caractérisé en ce qu'on fait agir une quantité efficace pour la régulation de la croissance des plantes d'un dérivé de cyclohexénone de formule Ic selon la revendication 4 ou un sel de Ic utilisable en agriculture ou un ester de Ic avec des acides carboxyliques en $C_1$-$C_{10}$ ou avec des acides inorganiques, sur des plantes, leur espace vital ou les semences.